(19) Europäisches Patentamt
European Patent Office
Office européen des brevets

(11) Publication number: 0 227 004
B1

(12) **EUROPEAN PATENT SPECIFICATION**

(45) Date of publication of patent specification: **11.04.90**

(51) Int. Cl.[5]: **C 07 C 331/28**, C 09 K 19/34, C 09 K 19/30, C 09 K 19/14, C 07 D 319/06, C 07 D 239/26

(21) Application number: **86117465.4**

(22) Date of filing: **16.12.86**

(54) **Liquid crystalline ethane derivatives, their preparation and the liquid crystal compositions containing same.**

(30) Priority: **16.12.85 PL 256832**

(43) Date of publication of application: **01.07.87 Bulletin 87/27**

(45) Publication of the grant of the patent: **11.04.90 Bulletin 90/15**

(84) Designated Contracting States: **CH DE FR GB IT LI**

(56) References cited:
**EP-A-0 126 883**
**EP-A-0 167 912**
**EP-A-0 169 327**
**EP-A-0 170 082**

**CHEMICAL ABSTRACTS, vol. 103, no. 8, 29th July-12th August 1985, page 547, column 1, abstract no. 30659j, Columbus, Ohio, US; R. DABROWSKI et al.: "Mesomorphic characteristics of some new homologous series with the isothiocyanato terminal group", & MOL. CRYST. LIQ. CRYST. 1985, 124(1-4), 241-257**

(73) Proprietor: **Wojskowa Akademia Techniczna im. Jaroslawa Dabrowskiego**
**PL-01-489 Warszawa-49 (PL)**
(73) Proprietor: **Zaklady Kineskopowe UNITRA POLKOLOR**
**Zaklad Kineskopow Kolorowych ul. Gen. St. Poplawskiego 7/9**
**PL-05-500 Piaseczno (PL)**

(72) Inventor: **Dabrowski, Roman**
**ul. Buska 40**
**Warszawa (PL)**
Inventor: **Dziaduszek, Jerzy**
**ul. Mlynarska 30a/26**
**Warszawa (PL)**
Inventor: **Szczucinski, Tomasz**
**ul. Rozlogi 9/4**
**Warszawa (PL)**
Inventor: **Drzewinski, Witold**
**ul. Graniczna 31**
**Blizne Laszcz (PL)**
Inventor: **Stolarz, Zofia**
**ul. Wilcza 14/30**
**Warszawa (PL)**
Inventor: **Zmija, Józef**
**ul. Mendelejewa 25**
**Warszawa (PL)**

**EP 0 227 004 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: Parka, Janusz
ul. Ciolka 24/39
Warszawa (PL)
Inventor: Sosnowska, Bozena
ul. Kartaginy 1/198
Warszawa (PL)

(74) Representative: von Füner, Alexander, Dr. et al
Patentanwälte v. Füner, Ebbinghaus, Finck
Mariahilfplatz 2 & 3
D-8000 München 90 (DE)

**Description**

The subject of the invention are liquid crystalline ethane derivatives, a method of their preparation and liquid-crystal compositions containing them.

The liquid crystalline materials known so far are applied in various displays as digital watches, calculators and so on. The scope of application of displays using liquid crystals is very rapidly increasing. At the moment they are also applied in various types of control instruments, in terminals of computers, in electronic games, in flat television screens and so on. They are also applied in instruments working in changing external conditions, as parking meters, pump meters at fuel stations, car dashboards, various types of portable instruments.

The development of liquid crystalline display applications requires liquid crystalline materials of improved properties ensuring reliable work of the display not only in closed rooms, where the working conditions change very little, and also in open-door conditions, where the temperature, humidity and ultraviolet radiation intensity greatly varies.

Liquid crystalline displays are usually constructed in such a way, that the layer of the liquid crystal of 7 to 11 μm thick is placed between two transparent, electrically insulated substrates (usually these are glass plates). An electrode structure is mounted on them enabling voltage application to the layer of the liquid crystal. The electrical voltage applied to the chosen element of the electrodes causes a change in the arrangement of the liquid crystal molecules in the layer, what in turn causes a visible optical effect in this region and is used in order to present information in the form of a series of letters, figures or graphs.

There is a large number of different effects which can result from reorientation of liquid crystal molecules by electric field. This is dependent on the type of the used liquid crystalline material as well as on the initial molecular arrangement of the liquid crystalline layer. The most frequently used effect in displays is the twisted nematic effect. In the twisted nematic effect, the liquid crystalline material is in its initial state arranged (electric field switched off) in parallel to the surface of the substrate through the action of the surface forces of the substrates in such a way, that when passing from the surface of one glass substrate to the surface of the second glass substrate, the long axis of the molecules are most frequently twisted by an angle of 90°. Such an arrangement is optically active, i.e. it rotates the plane of polarization of light. After applying a voltage larger than the threshold voltage, reorientating the molecules, the twist structures is destroyed and the liquid crystalline layer looses its optical activity. If a liquid crystalline layer arranged according to the presented rule is placed between the polarizers, it behaves as an optical valve, and is switched over by the field from the bright state to the dark one, depending on whether the two polarizers are connected in parallel with each other or perpendicularly.

If a dichroic dye, or a mixture of dyes, is placed in a liquid crystalline layer then switching over from a bright state to dark state or from a bright state to a coloured state or from a coloured state to a bright state, can be attained using one polarizer only. If we apply the cholesteric-nematic phase change effect and dichroic dyes, we do not require polarizers and the twist structure of the liquid crystalline layer in order to display. Liquid crystalline materials of positive dielectric anisotropy are required for the twisted nematic effect and the cholesteric-nematic phase change effect.

Static and dynamic electrooptical characteristics of liquid crystalline displays are related to dielectric anisotropy, optical anisotropy, elastic constants and viscosity of the liquid crystalline material. The basic problem, arising when applying nematic liquid crystalline materials in displays, is obtaining stable substances of low viscosity. Low viscosity of liquid crystalline material enables construction of fast operating displays. Especially advantageous are such materials which have a low dependence of viscosity from temperature, as liquid crystalline displays containing them will have reaction times hardly dependent on temperature.

No single liquid crystalline substance has characteristics fulfilling requirements demanded from liquid crystalline materials. According to the present art, a material of better performance in displays can be formed by mixing a few or a dozen or so of different compounds. Non liquid crystalline compounds and also compounds showing optical activity and dyes, can also be introduced to the material composed liquid crystalline compounds whose individual components are with nematic or sometimes smectic properties.

A mixture composed of appropriately chosen compounds satisfies the requirements of the liquid crystalline material for definite application more sufficiently. Such a mixture of course, has to be resistant to light, humidity, heat, oxygen and usually such compositions are preferred which have a low threshold voltage and a low supply voltage required for steering the display and which are characterized by a wide mesophase range. In order to obtain a liquid crystalline mixture of a wide mesophase range components are chosen in such a way as to form a basic composition of mesogenes of lowest melting point, to which 5—30% of tri- and tetra ring compounds are introduced. The basic mixture is formed from small double ring molecules most frequently being derivatives of azoxybenzene, biphenyl, phenylcyclohexane, phenylpyrimidine, phenyldioxane. Examples of such compounds are shown in patents: PL—A—107 551, GB—A—1 433 130, DE—A—2 636 684, DE—A—2 547 737, US—A—4 322 354, DD—A—139 852. The most frequently used compounds broadening the mesophase range are cyano derivatives of terphenyl, cyclohexylbiphenyl, diphenylpyrimidine or esters of cyclohexylbenzoic or biphenylic acids, the examples are given in patents: GB—A—1 433 130. DE—A—2 545 121. DE—A—2 701 591. DE—A—2 708 276 and DE—A—2 899 533. The above mentioned tri- or tetra ring liquid crystalline compounds applied as

components of the liquid crystalline mixture increase its clearing temperature but at same time their viscosity unfavourably increases. And so most favourable would be such compounds, which would broaden the mesophase range of the mixture with very slight changes in their viscosity.

Recently, multi ring non-polar hydrocarbons with dimethylene flexible bridges between the rings (M. Schadt, Mol. Cryst. Liq. Cryst., *34*, 138, [1983]) have been proposed in order to fulfil the above requirements. These compounds are characterized by small negative or positive dielectric anistropy and therefore their presence in the composition decreases its resultant positive anisotropy. This leads to an increase of threshold voltages of such mixtures. And so it would be more advantageous to apply compounds of similar characteristics concerning viscosity but having a large dielectric anisotropy.

A new class of liquid crystalline compounds 4-(trans-4′-n-alkyl cyclohexyl)benzenoisothiocyanates, which are characterized by very small viscosity, and low melting point, and allow to form basic compositions of viscosity about 10 cP, have been described in the US—A—4 528 116. However these compounds have a moderate anisotropy $\Delta E = 7 + 10$ and so the above mentioned hydrocarbons are less applicable for broadening the mesophase range. More advantageous would be compounds of larger positive dielectric anisotropy. Polar three-ring compounds having a terminal CN group are very slightly soluble in 4-(trans-4′-n-alkylcyclohexyl)benzeneisothiocyanate. Therefore they can be introduced into these compositions in very small concentrations. Compounds which would have better solubility with regard to liquid crystalline bi-ring isothiocyanates would be more appropriate.

According to the invention liquid crystalline compounds are new class of compounds having the required properties and they fulfil the above specified requirements. These are compounds of general formula 1:

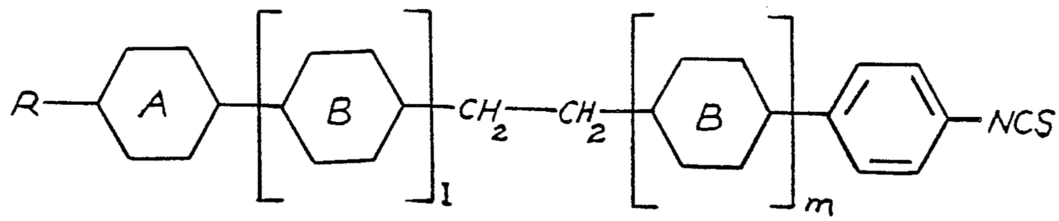

formula 1

where rings A and B are identical or different. When ring A indicates 1,4-substituted benzene ring

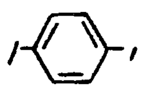

then ring B indicates 1,4-substituted benzene ring

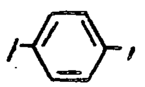

or 1,4-trans-substituted cyclohexane ring

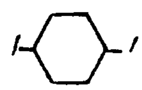

or 1,4-substituted bicyclo[2,2,2]octane ring

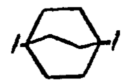

or 2,5-substituted pyrimidine ring

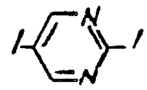

or 2,5-substituted 1,3-dioxane ring

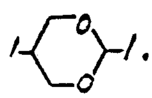

When ring B indicates 1,4-substituted benzene ring, then ring A indicates 1,4-trans-substituted cyclohexane ring or 2,5-substituted pyrimidine ring or 1,4-substituted bicyclo[2,2,2]octane ring or 2,5-substituted 1,3-dioxane ring.

R is a normal alkyl chain $C_nH_{2n+1}$, or a non-branched alkoxy group $C_nH_{2n+1}O$ or an alkylcarboxylic group $C_nH_{2n+1}COO$ or an alkylcarbonato group $C_nH_{2n+1}OCOO$ or a branched alkyl chain $CH_3—CH_2—CH(CH_3)—(CH_2)_k$ or a branched alkoxy group $CH_3—CH_2—CH(CH_3)—(CH_2)_k—O$, where n is an integer number and assumes values from 1 to 12, k is an integer number assuming values from 1 to 3, and l and m assuming values 1 or 0.

When l=0 and m=0, then the tetra ring compounds of general formula 1 are transformed into a bi-ring compound of general formula 1a:

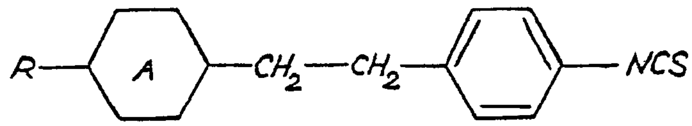

formula 1a

wherein ring A indicates only the 1,4-substituted bicyclo[2,2,]octane ring.

When l=0 and m=1, then the tetra ring compound of general formula 1 is transformed into a three ring compound of general formula 1b:

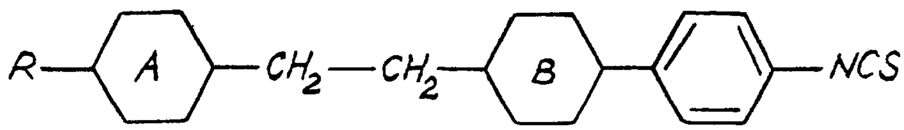

formula 1b

wherein, when ring A indicates 1,4-substituted benzene ring, then ring B indicates 1,4-substituted benzene ring or 1,4-substituted bicyclo[2,2,2]octane ring or 1,4-substituted cyclohexane ring or 2,5-substituted pyrimidine ring or 2,5-substituted 1,3-dioxane ring or when ring B indicates 1,4-substituted benzene ring, then ring A indicates 1,4-substituted bicyclo[2,2,2]octane ring or 2,5-substituted pyrimidine ring or 2,5-substituted 1,3-dioxane ring.

When l=1 and m=0, then the tetra ring compound of general formula 1 is transformed into a three ring compound of general formula 1c:

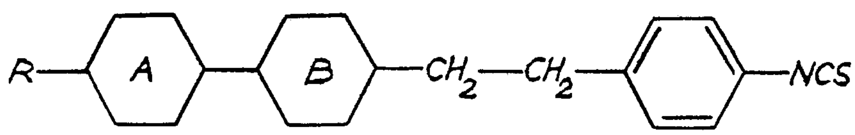

formula 1c

wherein, when ring A indicates 1,4-substituted benzene ring, then ring B indicates 1,4-substituted benzene ring or 1,4-substituted cyclohexane ring or 1,4-substituted bicyclo[2,2,2]octane ring or 2,5-substituted pyrimidine ring or 2,5-substituted 1,3-dioxane ring and when ring B indicates 1,4-substituted benzene ring, then ring A indicates 1,4-substituted cyclohexane ring or 1,4-substituted bicyclo[2,2,2]octane ring or 2,5-substituted pyrimidine ring or 2,5-substituted 1,3-dioxane ring.

According to the definition rings A and B and indicators 1 and m the following compounds are assigned to the general formula 1:

for l=0 and m=0

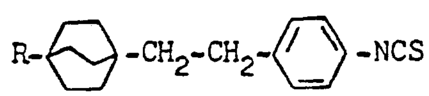

for l=0 and m=1

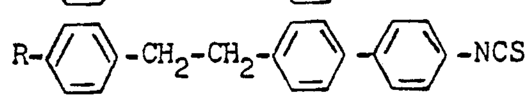

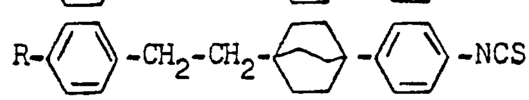

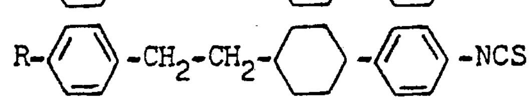

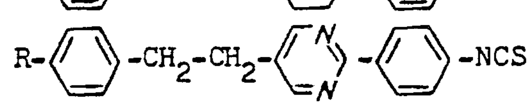

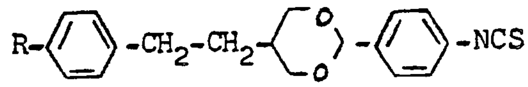

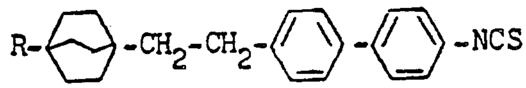

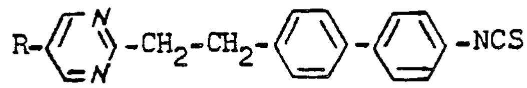

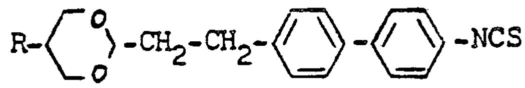

for l=1 and m=0

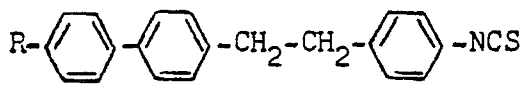

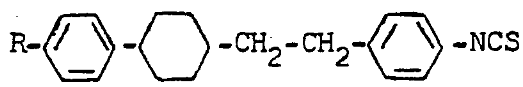

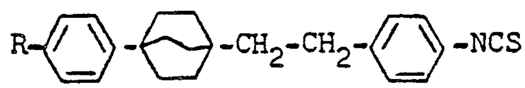

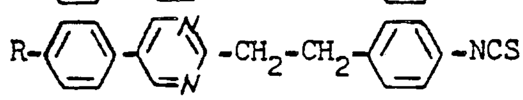

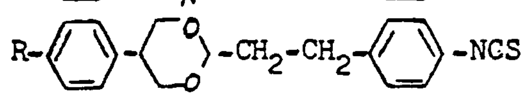

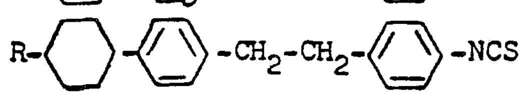

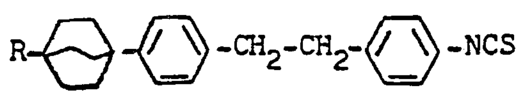

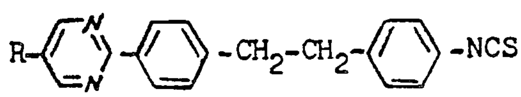

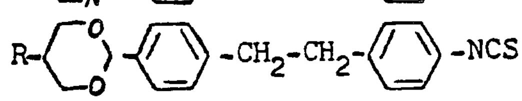

for l=1 and m=1

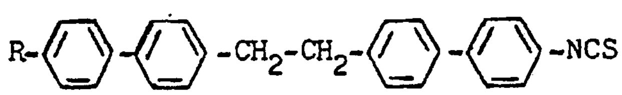

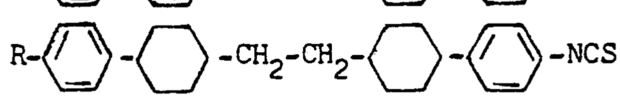

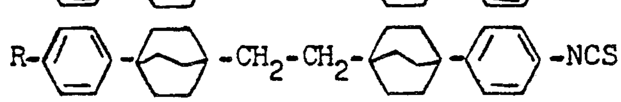

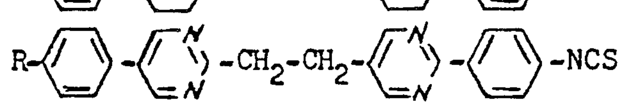

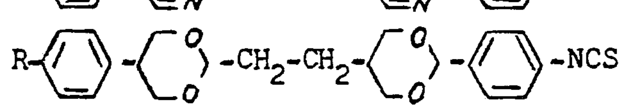

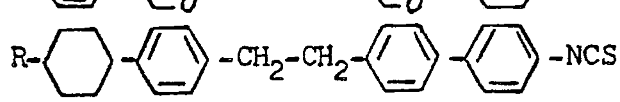

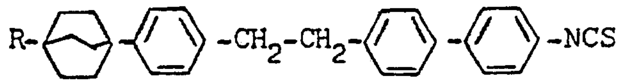

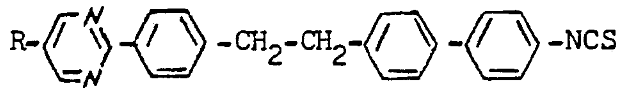

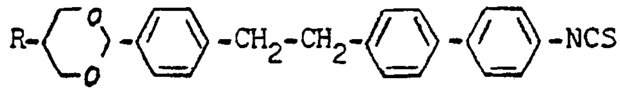

The preparation of liquid crystalline compounds of general formula 1 in the invention is specific in that the amine of formula 2:

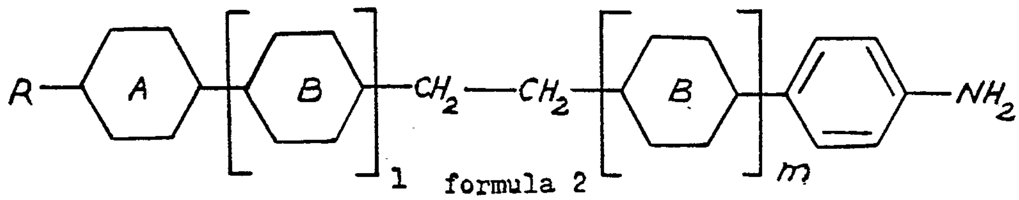

formula 2

in which the notations are the same as in formula 1 is treated according to scheme 1:

a) thiophosgene in the presence of an organic solvent preferably chloroform and a basic compound preferably calcium carbonate or sodium bicarbonate;

b) carbon disulfide in the presence of a solvent and a tertiary amine; the obtained trialkyloaminium dithiocarbamate is separated from the solution, and dissolved in an organic solvent and treated with an acid chloride in the presence of a tertiary amine; benzene is preferably applied as the solvent; triethylamine — as the tertiary amine, ethyl chloroformate — as the acid chloride;

c) carbon disulfide in the presence of an organic solvent preferably ether and dicyclohexylcarbodiimide.

As the result of all the different ways of preparing liquid crystalline compounds according to the invention the final product is separated by the known methods as evaporating or crystallization, or washing, drying, etc.

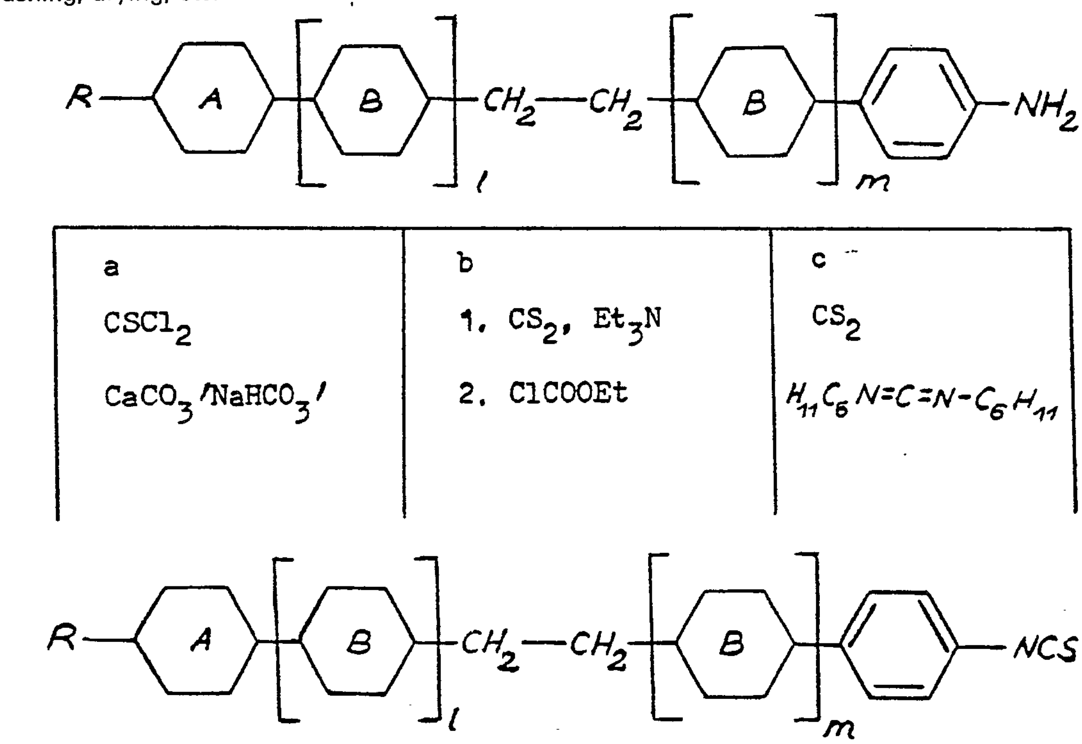


Scheme 1

Liquid crystalline compositions according to the invention is a liquid crystalline mixture including at least two liquid crystalline compounds, among which at least one component is the compounds of formula 1 in a quantity exceeding 2% by weight. The application of liquid crystalline compositions containing

5—40% by weight of compounds of formula 1 and other liquid crystalline compounds, optically active compounds and/or dyes is most advantageous.

The liquid crystalline display according to the invention contains two electrically isolated substrates (plates), of which at least one is transparent and electrode patterns are found on the internal surface of the substrates enabling the application of an electric field to the layer of the liquid crystalline composition containing at least one compound of formula 1.

Below are given examples of liquid crystalline compounds of formula 1, which are preferred as components of liquid crystalline compositions:

1-(p'-n-propylbiphenyl)-2-(p-isothiocyanatophenyl)ethane
1-(p'-n-butylbiphenyl)-2-(p-isothiocyanatophenyl)ethane
1-[p'-(2-methylbutyl)biphenyl]-2-(p-isothiocyanatophenyl)ethane
1-(p'-n-pentylbiphenyl)-2-(p-isothiocyanatophenyl)ethane
1-(p'-n-hexylbiphenyl)-2-(p-isothiocyanatophenyl)ethane
1-(p'-n-heptylbiphenyl)-2-(p-isothiocyanatophenyl)ethane
1-(p'-n-octylbiphenyl)-2-(p-isothiocyanatophenyl)ethane
1-(p'-n-pentyloxybiphenyl)-2-(p-isothiocyanatophenyl)ethane
1-[p'-(trans-4-n-propylcyclohexyl)phenyl]-2-(p-isothiocyanatophenyl)ethane
1-[p'-(trans-4-n-butylcyclohexyl)phenyl]-2-(p-isothiocyanatophenyl)ethane
1-[p'-(trans-4-n-pentylcyclohexyl)phenyl]-2-(p-isothiocyanatophenyl)ethane
1-[p'-(trans-4-n-hexylcyclohexyl)phenyl]-2-(p-isothiocyanatophenyl)ethane
1-[p'-(trans-4-n-heptylcyclohexyl)phenyl]-2-(p-isothiocyanatophenyl)ethane
1-[p'-(trans-4-n-octylcyclohexyl)phenyl]-2-(p-isothiocyanatophenyl)ethane
1-(p'-n-butylphenyl)-2-(p-isothiocyanatobiphenyl)ethane
1-(p'-n-pentylphenyl)-2-(p-isothiocyanatobiphenyl)ethane
1-(p'-n-hexylphenyl)-2-(p-isothiocyanatobiphenyl)ethane
1-(p'-n-butyloxyphenyl)-2-(p-isothiocyanatobiphenyl)ethane
1-[p'-(2-methylbutyloxy)phenyl]-2-(p-isothiocyanatobiphenyl)ethane
1-(p'-n-hexylphenyl)-2-[4-trans-(p-isothiocyanatophenyl)cyclohexyl]ethane
1-(p'-n-butylphenyl)-2-[4-(p-isothiocyanatophenyl)bicycloctyl]ethane
1-(p'-n-butylphenyl)-2-[2-(p-isothiocyanatophenyl)pyrimidyl-5]-ethane
1-(p'-n-butylphenyl)-2-[2-(p-isothiocyanatophenyl]-1,3-dioxyl-5]ethane
1-(4-n-butylbicyclooctyl)-2-(p-isothiocyanatobiphenyl)ethane
1-(5-n-butylpyrimidyl-2)-2-(p-isothiocyanatobiphenyl)ethane
1-(5-n-butyl-1,3-dioxyl-2)-2-(p-isothiocyanatobiphenyl)ethane
1-[p'-(5-n-propylpyrimidyl-2)phenyl]-2-(p-isothiocyanatophenyl)ethane
1-(p'-(5-n-butylpyrimidyl-2)phenyl]-2-(p-isothiocyanatophenyl)ethane
1-(p'-(5-n-hexylpyrimidyl-2)phenyl]-2-(p-isothiocyanatophenyl)ethane
1-[p'-(4-n-propylbicyclooctyl)phenyl]-2-(p-isothiocyanatophenyl)ethane
1-[p'-(4-n-butylbicyclooctyl)phenyl]-2-(p-isothiocyanatophenyl)ethane
1-[p'-(4-n-hexylbicyclooctyl)phenyl]-2-(p-isothiocyanatophenyl)ethane
1-[p'-(5-n-butyl-1,3-dioxyl-2)phenyl]-2-(p-isothiocyanatophenyl)ethane
1-[p'-(5-n-hexyl-1,3-dioxyl-2)phenyl]-2-(p-isothiocyanatophenyl)ethane
1-[trans-4-(p'-ethylphenyl)cyclohexyl]-2-(p-isothiocyanatophenyl)ethane
1-[trans-4-(p'-n-propylphenyl)cyclohexyl]-2-(p-isothiocyanatophenyl)ethane
1-[trans-4-(p'-n-butylphenyl)cyclohexyl]-2-(p-isothiocyanatophenyl)ethane
1-[trans-4-(p'-n-pentylphenyl)cyclohexyl]-2-(p-isothiocyanatophenyl)ethane
1-[trans-4-(p'-n-hexylphenyl)cyclohexyl]-2-(p-isothiocyanatophenyl)ethane
1-[trans-4-(p'-n-heptylphenyl)cyclohexyl]-2-(p-isothiocyanatophenyl)ethane
1-[trans-4-(p'-n-octylphenyl)cyclohexyl]-2-(p-isothiocyanatophenyl)ethane
1-[4-(p'-n-butylphenyl)bicyclooctyl]-2-(p-isothiocyanatophenyl)ethane
1-[4-(p'-n-pentylphenyl)bicyclooctyl]-2-(p-isothiocyanatophenyl)ethane
1-[4-(p'-n-hexylphenyl)bicyclooctyl]-2-(p-isothiocyanatophenyl)ethane
1-[5-(p'-n-propylphenyl)pyrimidyl-2]-2-(p-isothiocyanatophenyl)ethane
1-[5-(p'-n-butylphenyl)pyrimidyl-2]-2-(p-isothiocyanatophenyl)ethane
1-[5-(p'-n-hexylphenyl)pyrimidyl-2]-2-(p-isothiocyanatophenyl)ethane
1-[5-(p'-n-2-methylbutylphenyl)pyrimidyl-2]-2-(p-isothiocyanatophenyl)ethane
1-[5-(p'-n-butylphenyl)-1,3-dioxyl-2]-2-(p-isothiocyanatophenyl)ethane
1-[5-(p'-n-hexylphenyl)-1,3-dioxyl-2]-2-(p-isothiocyanatophenyl)ethane
1-(p'-n-butylbiphenyl)-2-(p-isothiocyanatophenyl)ethane
1-(p'-n-hexylbiphenyl)-2-(p-isothiocyanatobiphenyl)ethane
1-[p'-(trans-4-n-butylcyclohexyl)phenyl]-2-(p'-isothiocyanatobiphenyl)ethane
1-[p'-trans-4-n-hexylcyclohexyl)phenyl]-2-(p'-isothiocyanatobiphenyl)ethane
1-[p'-(5-n-butylpyrimidyl-2)phenyl]-2-(p'-isothiocyanatobiphenyl)ethane
1-[p'-(5-n-butylpyrimidyl-2)phenyl]-2-(p-isothiocyanatophenyl)ethane
1-[p'-(4-hexylbicyclooctyl)phenyl]-2-(p-isothiocyanatobiphenyl)ethane

1-(p'-n-butyloxybiphenyl)-2-(p-isothiocyanatobiphenyl)ethane
1-[trans-4-n-butylphenyl)cyclohexyl]-2-[trans-4-(p-isothiocyanatophenyl)cyclohexyl]ethane
1-[trans-4-(p-n-hexylphenyl)cyclohexyl]-2-[trans-4(p-isothiocyanatophenyl)cyclohexyl]ethane
1-[5-(p'-n-butylphenyl)pyrimidyl-2]-2-[2-(p-isothiocyanatophenyl)pyrimidyl-5]ethane
1-[5-(p'-n-hexylphenyl)pyrimidyl-2]-2-[2-(p-isothiocyanatophenyl)pyrimidyl-5]ethane

The above-mentioned compounds with an alkyl chain containing an even number of carbon atoms especially compounds with a butyl or hexyl radical are escpecially advantageous among the preferred compounds.

Compounds of formula 1 are especially advantageous components of the liquid crystalline compositions containing known liquid crystalline compounds with the isothiocyanate group.

An example of such compounds are compounds in formulae I—VII.

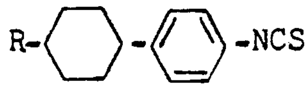
I

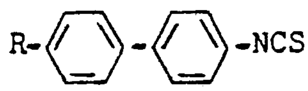
II

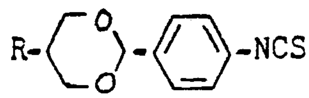
III

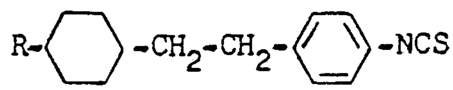
IV

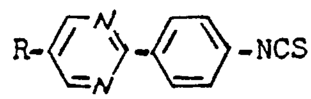
V

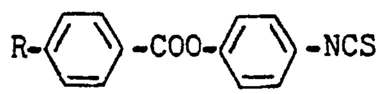
VI

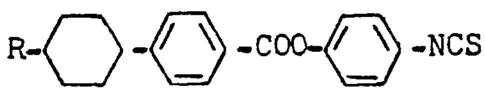
VII

where R is a normal or branched alkyl group containing from 3 to 10 carbon atoms in the alkyl chain.

Compounds of formula 1 dissolve easily in other liquid crystalline compounds, especially in mixtures obtained from known compounds of type 4-(trans-4'-n-alkylcyclohexyl)-1-isothiocyanatobenzenes as 4-(trans-4'-n-propylcyclohexyl)-1-isothiocyanatobenzene or 4-(trans-4'-n-hexylcyclohexyl)1-isothiocyanatobenzene expressed by formula I and 4-alkyl-4-isothiocyanatobiphenyls expressed by formula II, and then form a composition without segregation or separation of components of higher melting point especially at low temperatures. They increase the clearing point of low nematic-isotropic transition temperature compositions to the range being sufficient for practical application, with only small increasing their viscosity. The response times of displays with the compositions including compounds of formula 1 are hardly dependent in temperature.

Compounds of formula 1 according to the invention can also be applied as components of liquid crystalline mixtures composed simultaneously of known liquid crystalline compounds, the molecules of which have a terminal isothiocyanate group and compounds, the molecules of which do not have a terminal isothiocyanate group, but have other terminal groups such as cyano, alkyl and alkoxy groups. An example of such liquid crystalline compounds are compounds of formula VIII—XXIV, and they are given below:

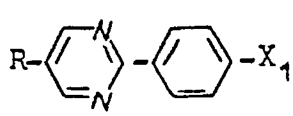
VIII

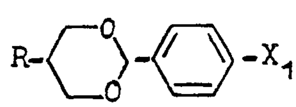
IX

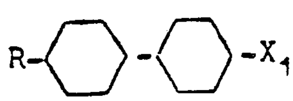
X

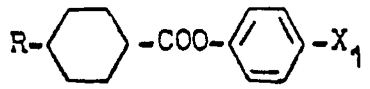
XI

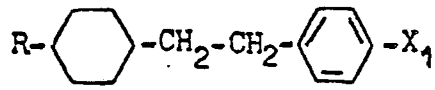
XII

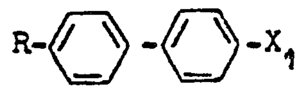
XIII

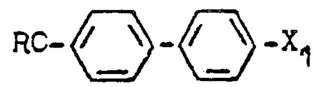
XIV

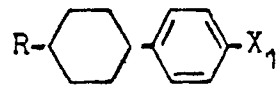
XV

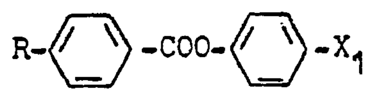
XVI

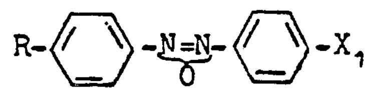
XVII

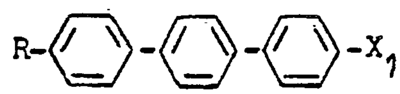
XVIII

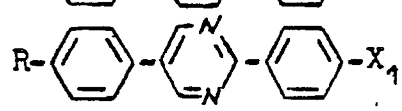
XIX

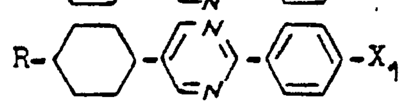
XX

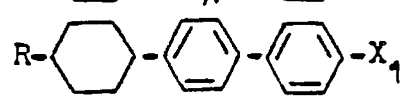
XXI

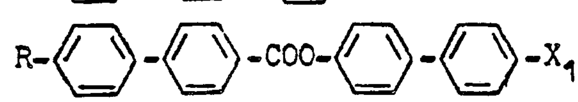
XXII

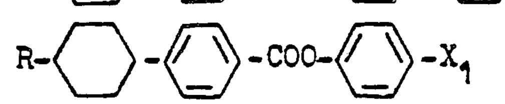
XXIII

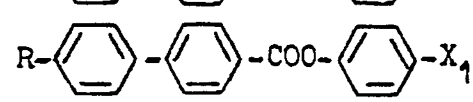
XXIV

In formulae VIII—XIV R indicates an alkyl group and $X_1$ is the cyano group (CN) or the alkyl group or the alkoxy group. With respect to the above mentioned compounds, the most advantageous ones are those characterized by a large positive dielectric anisotropy.

Compounds of formula 1 can also be applied as components of liquid crystalline compositions containing known compounds, with remaining components except of compounds of formula 1, do not have an isothiocyanate group as the terminal one. Liquid crystalline compositions obtained according to this invention are characterized by a very good solubility of dichroic dyes and they have especially good performance in the application of the effect guest-host for colour display.

Amines of formula 2 being the substrate for preparing compounds 1 of this invention can be obtained in numerous ways:

*Method 1,* which is a general method of obtaining compounds of formula 2, for cases when m=0 is shown in scheme 2.

Method 1.

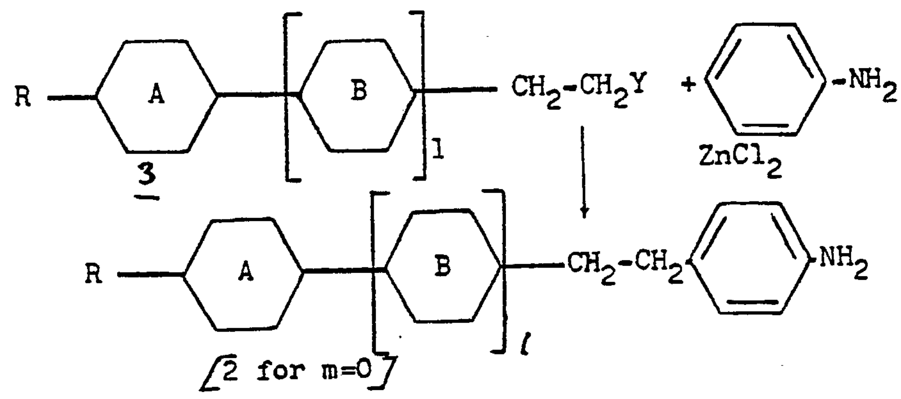


Scheme 2

In this method compound 3, which is β-substituted ethanol or its halogen derivative is heated with aniline in the presence of $ZnCl_2$, similarly as it has been described in the Polish patent No 124624 for obtaining p-alkylaniline. The substrate 3 may be obtained from compounds 4 according to the procedure given by Zytyński in Mol. Cryst. Liq. Cryst., *87,* 109 (1982) for p-pentyl-p'-(2-chloroethyl)biphenyl.

*Method 2* shown in scheme 3 concerns compounds of formula 2, in which ring B is the benzene ring and m=0 or m=1.

Method 2

Step 1

Step 2

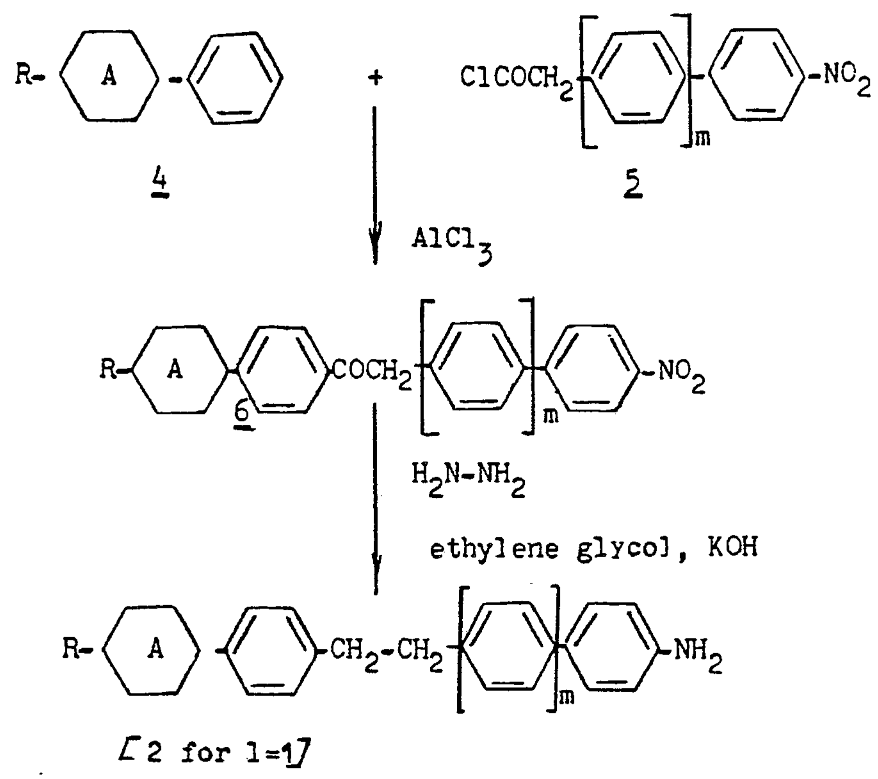


Scheme 3

The substrate 4 is easily obtained according to the method described by Dabrowski in Mol. Cryst. Liq. Cryst., *58,* 251, (1980), when ring A is the benzene ring or in Mol. Cryst. Liq. Cryst., *85,* 55 (1982), when A is the cyclohexane ring, or by Gray's in J. C. S. Perkin II, 1981, 26, when A is the bicyclooctane ring, or by Boller in Mol. Cryst. Liq., *42,* 215 (1977) when A is the pyrimidine ring or by Vorbrodt in J. Pract. Chem., *323,* 902, (1981) when A is the 1,3-dioxane ring.

The second substrate, compound 5, may be obtained by nitration of phenyl acetic or biphenylacetic acid or benzyl cyanide by the method described in Practical Organic Chemistry, A. Vogel, WNT 1984, p. 536.

In the first step compound 5 reacts with the phenyl acetic or biphenyl acetic chloride giving the keto derivative 6, which in the second stage, in the conditions of the Wolff-Kishner reaction is simultaneously reduced on the nitro group and the carbonyl group.

The remaining methods 3,4,5,6 are especially advantageous for preparing some compounds of formula *2.*

*Method 3* can be applied for preparing compound 2 when ring A is the benzene ring and ring B is the pyrimidine ring and m=0.

Method 3

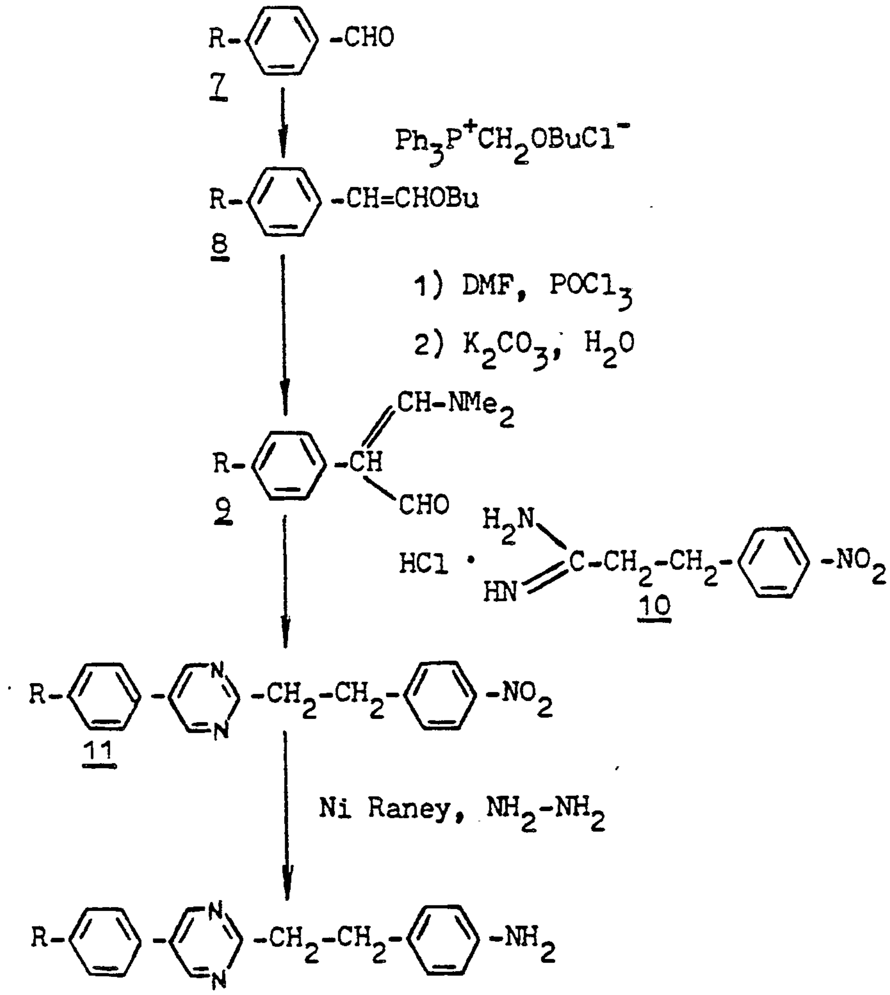


Step 1

Step 2

Step 3

Step 4

7

8

9

10

11

(2 for A=benzene ring, B=pyrimidine ring and m=0)

Scheme 4

Compound *7* has been obtained by the Smith method, J. Org. Chem., *37,* 3972 (1972) and then used in the first step of the reaction conducted according to G. Wittig and M. Schlosser, Chem. Ber., *94* 1373 (1961). Step 2 has been conducted in a similar way to Z. Arnold, Coll. Czech. Chem. Comm., *26,* 305 (1961). Condensation of N-dimethyloacrolein *9* with amidine *10* was conducted according to Zaschke, J. Prac. Chem., *317,* 617 (1979), and the obtained compound *11* was reduced with hydrazine by a standard method in the presence of Raney nickel.

*Method 4* is specific for obtaining compounds of formula *2,* where ring A indicates the benzene ring and ring B indicates the dioxane ring and m=0.

Method 4

Step 1

Step 2

Step 3

Step 4

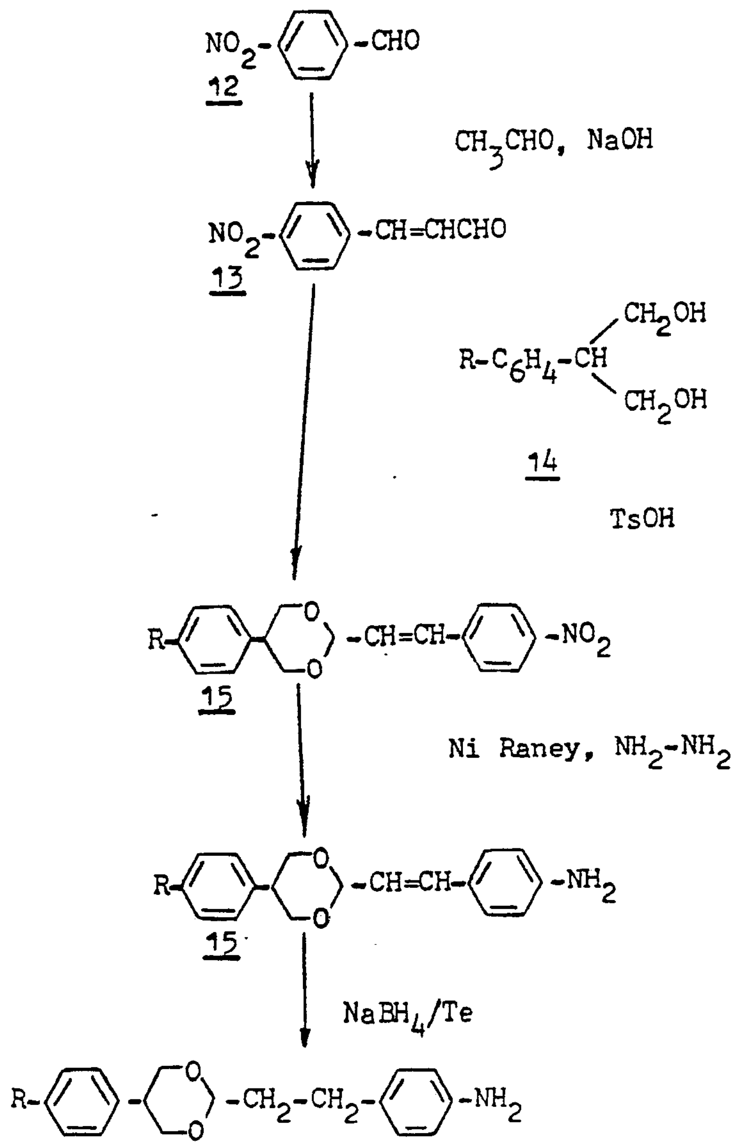


(2 for A=benzene ring, B=dioxane ring, m=0)
Scheme 5

The first step of this method was conducted according to T. Nishimura, Bull. Chem. Soc. Jap., *25*, 54 (1952); condensation with alkylphenylpropanediol-1,3 (compound 14) and reduction of the nitrate group (steps 2 and 3) was conducted by the method described in Mol. Cryst. Liq. Cryst., 124, 241 (1985). The reduction of the double bond was conducted according to the K. Romasamy method, Synthesis, 545 (1978).

*Method 5* is a more general method for obtaining compounds 2, containing the pyrimidine ring.

Method 5

Step 1

Step 2

Step 3

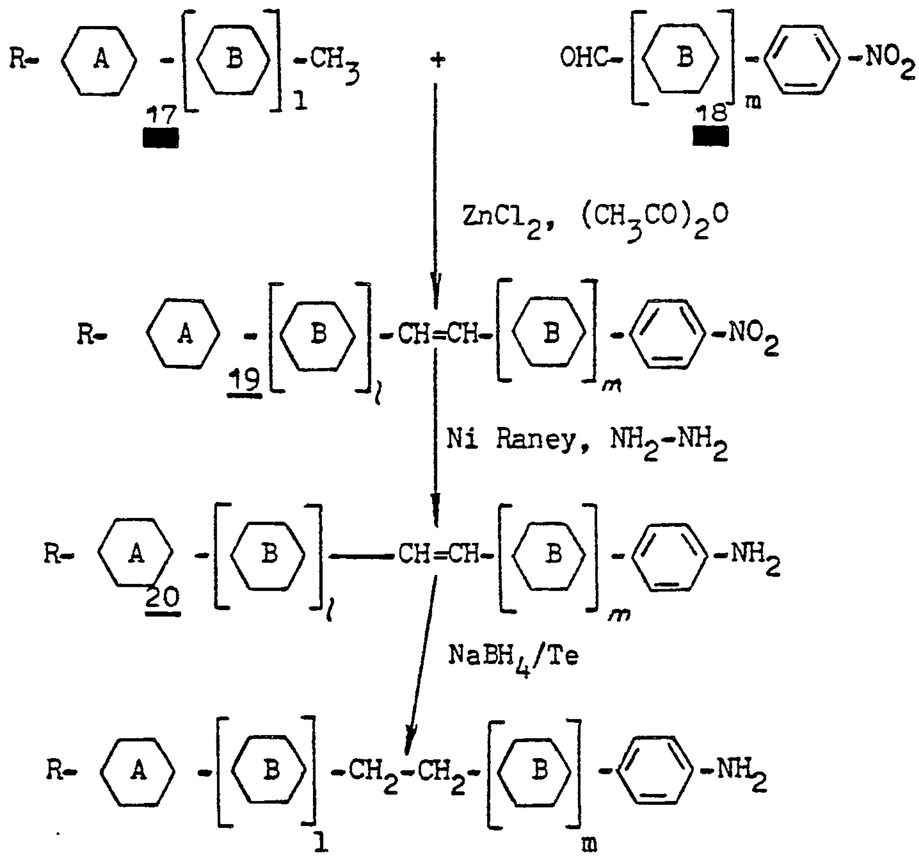


Scheme 6

Method 5 shown in scheme 6, is especially advantageous for compounds *2*, where ring B is the pyrimidine ring l=1, m=1, or where ring A is the pyrimidine ring and l=0, m=1 or 0. In method 5 the methyl pyrimidine derivative (compound 17) was heated in acetic anhydride in the presence of anhydrous zinc chloride and stilbene derivative was obtained (compound 19) and reduced to the amino derivative (compound (2) by an analogous method to method 4 (stages 3 and 4).

*Method 6* is a more general method of obtaining compounds 2 containing the dioxane ring.

Method 6

Step 1

Step 2

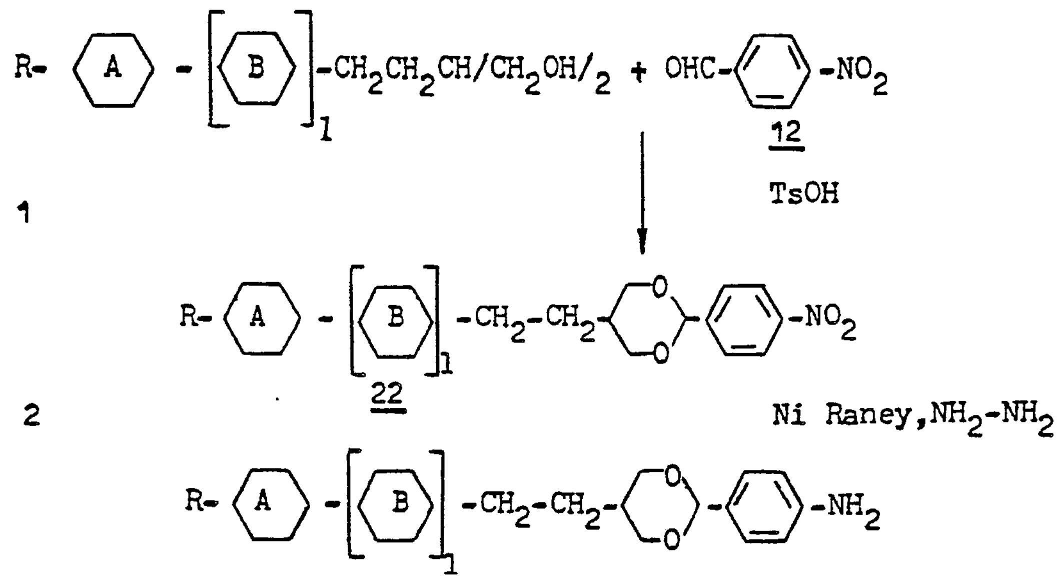


(compound 2, ring B=dioxane ring, m=1)
Scheme 7

This method is especially preferred for obtaining compounds 2, where l=0. Stage 1 was conducted by heating the propanediol-1,3-derivative (compound 21) with p-nitrobenzoic aldehyde. The nitro group in compound *22* was reduced with hydrazine in the presence of Raney nickel by the method described in Mol. Cryst. Liq. Cryst., *124*, 241 (1985).

Compounds of the above invention, expressed by formula 1 will be illustrated in details below by examples showing the method of preparation compounds expressed by formula *1* and also the method of preparation the essential starting compounds; especially the method of obtaining compounds of formula *2* will be shown in details. Also the characteristic features of compounds thus obtained and characteristic features of liquid crystalline compositions containing compounds of formula *1* will be discussed in the examples. The enclosed examples given below are given in order to illustrate the scope of this invention, however, without limiting the range of the invention. The used symbols indicate:
Cr — solid phase, S — smectic phase, N — nematic phase, I — isotropic phase.

Example 1

1-[p'-(trans-4-n-heptylcyclohexyl)phenyl]-2-(p-isothiocyanatophenyl)ethane

To a mixture 50 $cm^3$ of water, 50 $cm^3$ of chloroform, 4 g of calcium carbonate and 3,5 g (0,03 moles) of thiophosgene, a solution 10 g of (0,026 moles) 1-[p'-(trans-4-n-heptylcyclohexyl)phenyl]-2-(p-aminophenyl)ethane in 50 $cm^3$ of chloroform was added dropwise at room temperature and the vigorously stirred. It was further stirred for two hours, and then a small quantity of 5% hydrochloric acid (20 $cm^3$) was added dropwise and the two layers were separated. The chloroform layer was washed with water and dried over $MgSO_4$. After the chloroform was distilled off the solid residue was recrystallized from isopropanol using active carbon for decolouring and then twice from n-hexane and once from a mixture of methanol-chloroform. 3 g (27% yield) of 1-[p'-(trans-4-n-heptylcyclohexyl)phenyl]-2-(isothiocyanatophenyl)ethane was obtained with the temperatures of phase transition Cr 53,5 $Cr_1$71 N 118 I.

The following compounds were obtained in a similar way:

1-[p'-(trans-4-n-propylcyclohexyl)phenyl]-2-(p-isothiocyanatophenyl)ethane. Temperatures of phase transition Cr 107 N 120 I.

1-[p'-(trans-4-n-hexylcyclo)phenyl]-2-(p-isothiocyanatophenyl)ethane. Temperatures of phase transition Cr 58 N 136 I.

1-(p'-n-pentylbiphenyl)-2-(p-isothiocyanatophenyl)ethane. Temperatures of phase transition Cr 96,5 N 141 I.

1-(p'-n-hexylbiphenyl)-2-(p-isothiocyanatophenyl)ethane. Temperatures of phase transition Cr 56 $S_1$ 99,5 N 132,5 I.

1-(p'-n-heptylbiphenyl)-2-(p-isothiocyanatophenyl)ethane. Temperatures of phase transition Cr 70 S 103 N 135 I.

1-[p'-(2-methylbutyl)biphenyl]-2-(p-isothiocyanatophenyl)ethane. Temperatures of phase transition Cr 71,5 S 98 I.

1-(trans-4-n-pentylcyclohexyl)-2-(p-isothiocyanatophenyl)ethane. Temperatures of phase transition Cr 95,5 N 190 I.

1-(p-n-pentylphenyl)-2-[trans-4-(p-isothiocyanatophenyl)cyclohexyl]ethane. Temperatures of phase transition Cr 68,5 N 119 I.

1-(p'-n-pentyloxybiphenyl)-2-(p-isothiocyanatophenyl)ethane. Temperatures of phase transition Cr 63 S 129—130 N 164,5 I.

Example 2

1-(p-n-butylphenyl)-2-[2-trans-(p'-isothiocyanatophenyl)-1,3-dioxyl-5]ethane.

A mixture of 11,2 g of $CaCO_3$, 50 $cm^3$ of water, 35 $cm^3$ of chloroform, 8,4 g (0,078 mole) of thiophosgene was cooled to 0°C and at this temperature a solution of 19 g (0,056 mole) of 1-(-p-n-butylphenyl)-2-2-p-aminophenyl)-1,3-dioxyl-5 ethane in 100 $cm^3$ of chloroform was added dropwise while stirring. The mixture was stirred for four hours, and then the layers were separated. The chloroform layer was washed with water and dried over anhydrous $MgSO_4$, filtered through silica gel. After the chloroform was evaporated the solid product was recrystallized from a methanol-tetrahydrofuran (1:4) method and then from hexane. 10 g (47% theor yield) of 1-(p-n-butylphenyl)-2-[trans-2-(p'-izothiocyanatophenyl)-1,3-dioxyl-5]ethane was obtained. The temperatures of phase transition Cr 94 N 113 I.

The following compounds were obtained in a similar way: 1-(p-n-pentylphenyl)-2-[trans-2(p'-isothiocyanatophenyl)-1,3-dioxyl-5]ethane. Temperatures of phase transition Cr 92 N 118 K.

Example 3

1-[p'-n-hexylbiphenyl)-2-(p-isothiocyanatophenyl)-ethane 18 g (0,05 moles) 1-(p'-n-hexylbiphenyl)-2-(p-aminophenyl)ethane was dissolved in 200 $cm^3$ of benzene and 25 $cm^3$ of hexane. Then 14 $cm^3$ (0,1 moles) of triethylamine and 6 $cm^3$ (0,1 moles) carbon disulphide was added to the solution. The components were well stirred and left in the fridge for 48 hours. A yellow salt of triethyloammonium ditiocarbamate was precipitated and filtered off and then washed with ether. The obtained product was dissolved in 100 $cm^3$ of chloroform, 10,5 $cm^3$ of triethylamine was added to the solution and after cooling to 0°C 7 g (0.075 mole) of

ethyl chloroformate was added dropwise to the constantly stirring solution. After an hour 100 $cm^3$ of 3N hydrochloric acid was poured into the reaction mixture, the layers were separated and the chloroform one was washed twice with water and dried over $MgSO_4$. After distilling chloroform off the solid residue was crystallized from isopropanol, then twice from n-hexane and once from a composition of methanol-chloroform. 5 g of (25% yield) 1-(p'-n-hexylbiphenyl)-2-(p-isothiocyanatophenyl)ethane was obtained. The temperatures of phase transition Cr 56 $S_1$ 99,5 N 133 I.

Example 4

1-(p'-n-heptylbiphenyl)-2-(p-isothiocyanatophenyl)ethane

10,2 g (0,05 mole) of dicyclohexylcarbodiimide (DCC) was dissolved in 50 $cm^3$ of dry ether and then 60 g of carbon disulphide, a solution of 16,7 g (0,945 mole) of 1-(p'-n-heptylbiphenyl)-2-p-aminophenyl)ethane in 50 $cm^3$ of ether were added to the solution. The mixture was left for a few hours and then crystals of dicyclohexyltiourea were filtered, the filtrate was concentrated, the residue was dissolved in benzene and then filtered through a layer of silica gel. The filtrate was concentrated until dry and the solid was recrystallized from isopropanol and then twice from n-hexane. 4 g (21% yield) of 1-(p'-n-heptylbiphenyl)-2-(p-isothiocyanatophenyl)ethane was obtained. The temperatures of phase transition are Cr 70 S 103 N 135 I.

Example 5

1-(p'-n-hexylbiphenyl)-2-(p'-aminophenyl)ethane

Step 1

A solution containing 52,4 g (0,26 mole) of p-nitro phenylacetic acid chloride in 300 $cm^3$ methylene chloride was added dropwise, at room temperature, to a stirring mixture composed of 40 g (0,3 mole) anhydrous aluminium chloride and 200 $cm^3$ of methylene chloride. Then the mixture was cooled to 0°C and at this temperature a solution 59,5 g (0,25 mole) p-hexylbiphenyl in 200 $cm^3$ methylene chloride was added dropwise. The reaction mixture was stirred for 4 hours at 0°C and then it was poured out into water with ice. The layers were separated. The organic layer was washed four times with water and dried over anhydrous $MgSO_4$ and the solvent was distilled off under vacuum. The raw product was recrystallized from acetone giving 71 g (70,8% yield) of 4-hexyl-4'-(p-nitrophenylacetyl)biphenyl with melting point 132—133°C.

The following compounds were obtained in a similar way:

4-n-pentyl-4'-(p-nitrophenylacetyl)biphenyl, melting point 133—134°C.

4-n-heptyl-4'-(p-nitrophenylacetyl)biphenyl, melting point 133—134°C.

4-n-pentyloxy-4'-(p-nitrophenylacetyl)biphenyl, temperatures of phase transition: Cr 151—152 N 156,5 I.

4-(2-methylbutyl)-4'-(p-nitrophenylacetyl)biphenyl, melting point 103—104°C.

4-[trans-4-(p'-nitrophenyl)cyclohexylacetyl]-n-pentylbenzene, melting point 100—101°C.

Step 2

A mixture containing 64 g (0,16 mole) of 4-hexyl-4'-(p-nitrophenylacetyl)biphenyl, 50 g (0,8 mole) 80% hydrazine hydrate and 700 $cm^3$ of diethylene glycol was heated to 140—145°C for 6 hours. Then it was cooled to room temperature and 27 g (0,48 mole) of potassium hydroxide was added and again heated distilling off volatile components until a temperature 200°C was reached. Then it was boiled under reflux condenser at 200°C for 4 hours. After cooling the reaction mixture was diluted with water. The precipitated amine was extracted with toluene (1,51), the toluene solution was filtered through a layer of active carbon and dried over $MgSO_4$. The solvent was distilled off and the residue recrystallized from isopropanol. 25 g of 1-(p'-hexylbiphenyl)-2-(p-aminophenyl)ethane (43% yield) was obtained with melting point 107—110°C.

Example 6

1-(p'-n-pentylbiphenyl)-2-(p-aminophenyl)ethane

18.6 g (0,2 mole) of distilled aniline, 57,4 g (0,2 mole) of 1-(p-n-pentylbiphenyl)-2-chloroethane, 20,4 g of remelted $ZnCl_2$ were heated for 10 hours at 220°C. Then the mixture was cooled to 80°C, 200 $cm^3$ of ethanol was added and the lot was boiling under reflux for 4 hours. White crystalline precipitate was filtered off and heated for 2 hours with 200 $cm^3$ of 10% aqueous NaOH solution. The separated oil substance was extracted with toluene, the extract was dried over solid KOH and toluene was distilled off. The residue was recrystallized from isopropanol. 26,5 g (35% yield) of 1-(p-n-pentylbiphenyl)-2-(p-aminophenyl)ethane was obtained, melting point 110—113°C.

Example 7

1-[p-(trans-4-heptylcyclohexyl)phenyl]-2-(p'-aminophenyl)ethane

Step 1

30 g (0,15 moles) of p-nitrophenyl acetic acid chloride dissolved in 150 $cm^3$ of methylene chloride was added dropwise to a stirred mixture of 200 $cm^3$ of methylene chloride and 25,7 g (0,195 mole) of anhydrous aluminium chloride. Then it was cooled to 0°C and at this temperature a solution containing 38,8 g (0,15 mole) of 4-heptylcyclohexylbenzene with about 70% of the trans isomer and 150 $cm^3$ of methylene chloride was added dropwise. The reaction mixture was stirred for 4 hours a 0°C and then poured into water with

ice. The organic layer was separated, washed with water and dried over $MgSO_4$. Methylene chloride was distilled off and the residue recrystallized from acetone, 23 g (36% yield) of 4-(p-nitrophenylacetyl)-1(trans-4-heptylcyclohexyl)benzene was obtained, melting point 145—146°C.

The following compounds were obtained in a similar way:

4-(p-nitrophenylacetyl)-1-(trans-4'-n-propylcyclohexyl)benzene melting point 166—168°C.

4-(p-nitrophenylacetyl)-1-(trans-4'-n-hexylcyclohexyl)benzene melting point 143—145°C.

Step 2

A mixture of 22 g (0,05 mole) 4-(p-nitro-phenylacetyl)-1-(trans-4-heptylcyclohexyl)benzene and 13 g (0,25 mole) of 80% hydrazine hydrate and 200 $cm^3$ diethylene glycol was heated for 6 hours at 140—145°C. Then 8 g (0,15 mole) of KOH was added and the mixture was further heated to 210°C, then it was cooled, diluted with water and extracted with toluene. The toluene extract was washed with water, filtered through a layer of active carbon, dried over $MgSO_4$. The solvent was distilled off and the solid residue was recrystallized from isopropanol giving 11 g (56% yield) of 1-[p-trans-4-heptylcyclohexyl)phenyl]-2-(p-aminophenyl)ethane, melting point 110—115°C.

The following compounds were obtained in a similar way:

1-[p-(trans-4-n-propylcyclohexyl)phenyl]-2-(p'-aminophenyl)ethane, melting point 130—131°C.

1-[p-(trans-4-n-hexylcyclohecyl)phenyl]-2-(p'-aminophenyl)ethane, melting point 117—120°C.

Example 8

1-(p-n-butylphenyl)-2-[2-trans-(p'-aminophenyl)-1,3-dioxyl-5]ethane

Stage 1

A mixture of 28,3 g (0,12 mole) of 2-[2-(p-n-butylophenyl)ethyl]propane-diol-1,3, 20 g (0,13 mole) of p-nitrobenzoic aldehyde, 0,5 g of p-toluenesulfonic acid, 200 $cm^3$ of benzene was heated under Dean-Stark trap until water evolved. Then the mixture was washed with $Na_2CO_3$ solution, water and dried over anhydrous $MgSO_4$. Benzene was distilled off, the solid residue was crystallized twice from isopropanol, 32 g (72% yield) of 1-(p-n-butylphenyl)-2-[2-trans-(p'-nitrophenyl)1,3-dioxyl-5]ethane was obtained, melting point 96—98°C. The following compounds were obtained in a similar way: 1-(p-n-pentylphenyl)-2-[trans-(p'-nitrophenyl)-1,3-dioxyl-5]ethane, melting point 84—87°C.

Step 2

A mixture of 30 g of 1-(p-n-butyl phenyl)-2-[2-trans-(p-nitrophenyl)-1,3-dioxyl-5]ethane, 10,5 of hydrazine hydrate, 200 $cm^3$ of methanol was heated to 45°C and then Raney nickel was gradually added until temperature stopped increasing. Then it was heated at refluxing for an hour, Raney nickel was filtered off and the solution cooled. 10 g (66,7% yield) of 1-(p-n-butylphenyl)-2-[2-trans-(p'-aminophenyl)1,3-dioxyl-5]ethane was obtained melting point 99—103°C.

Example 9

1-[5-(p'-n-butylphenyl)pyrimidyl-2]-2-(p-nitrophenyl)ethene

A mixture of 4,0 g (0,017 mole) of 5-(p-n-butylphenyl)-2-methylpyrimidine, 4,0 g (0,026 mole) of p-nitrobenzoic aldehyde, 1 g of ZnCl, 10 $cm^3$ of acetic anhydride was refluxed for 6 hours, then the reaction mixture was poured onto water with ice and extracted with 250 $cm^3$ of benzene. The extract was dried over $MgSO_4$, and benzene was distilled off. The residue was recrystallized from a mixture of methanol-tetra-hydrofurane (3:1) and twice from ethanol 1 g (16% yield) of 1-[5-(p'-n-butylphenyl)pyrimidyl-2]-2-(p-nitrophenyl)ethene was obtained, temperatures of phase transition Cr 170 $S_A$ 202 N 245 I.

Example 10

A composition in % by weight.

4-(trans-4'-n-propylcyclohexyl)-1-isothiocyanatobenzene-26

4-(trans-4'-n-hexylcyclohexyl)-1-isothiocyanatobenzene-27,3

4-(trans-4'-n-octylcyclohexyl)-1-isothiocyanatobenzene-11,7

4-pentyl-4'-isothiocyanatobiphenyl-15

1-(p-n-heptylbiphenyl)-2-(p'-isothiocyanatophenyl)ethane-10

1-p-(trans-4-n-heptylcyclohexyl)phenyl-2-(p'-isothiocyanatophenyl)ethane-10

is a nematic in the temperature range from (−35°C) to 59°C. At 20°C it is characterized by the following parameters: viscosity, $\eta = 12$ mPa·S, dielectric anistrotropy, $\Delta\varepsilon = \varepsilon_{\parallel} - \varepsilon_{\perp} = +9$, optical anisotropy, $\Delta n = n_e - n_o = 0{,}22$.

The liquid crystalline cell of thickness 10 µm filled by this composition has the following electrooptical parameters:

| | Temperature | | | |
|---|---|---|---|---|
| | −20°C | 0°C | 20°C | 40°C |
| Threshold voltage, $V_{10\%}$ (Volt) | 2,0 | 1,8 | 1,2 | 1,5 |
| Saturation voltage $V_{90\%}$ (Volt) | 3,0 | 2,8 | 2,7 | 2,4 |
| Rise time, $T_{on}$ 10—90% (ms) | 300 | 100 | 95 | 90 |
| Decay time $T_{off}$ 90τ 10% (ms) | 300 | 170 | 160 | 160 |

Example 11

0,4% by weight of a blue dichroic dye of formula (given below) was added to the composition of content given in example 10.

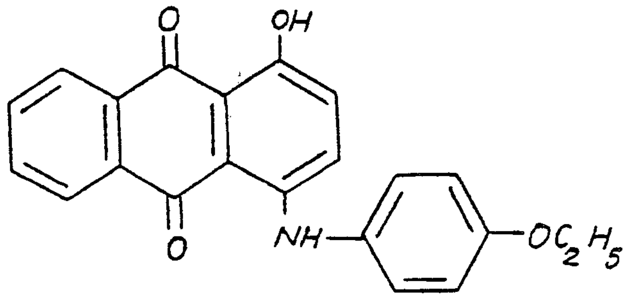

A high display contrast, dichroic ratio CR=10, and order parameter S=0,75 were observed for this composition.

Example 12

A composition of the following content was prepared:

| | |
|---|---|
| Composition from example 10 | 97% by weight |
| Optically active 5-(2-methylbutyl)-2-isothiocyanatophenyl-1,3-dioxane | 3% by weight |

This composition shows the cholesteric-nematic phase transition for voltage 4 V, at 20°C, and the reverse nematic-cholesteric transition is observed for voltage 1 V.

Example 13

A composition in % by weight:

| | |
|---|---|
| 4-(trans-4'-n-propylcyclohexyl)-1-isothiocyanatobenzene | 25,9 |
| 4-(trans-4'-n-hexylcyclohexyl)-1-isothiocyanatobenzene | 27,2 |
| 4-(trans-4'-n-octylcyclohexyl)-1-isothiocyanatobenzene | 11,6 |
| 5-n-pentyl-2-(p-cyanophenyl)pyrimidine | 5,0 |
| 1-[p'-(trans-4-n-heptylcyclohexyl)phenyl)-2-(p-isothiocyanatophenyl)ethane | 9,9 |
| 1-(p'-n-hexylbiphenyl)-2-(p-isothiocyanatophenyl)ethane | 10,0 |
| 5-(p-n-butylphenyl)-2-(p'-cyanophenyl)pyrimidine | 5,0 |
| 5-(trans-4-pentylcyclohexyl)-2-(p-cyanophenyl)pyrimidine | 5,0 |
| optically active p-(2-methylbutyl)-p'-cyanobiphenyl | 0,4 |

is a nematic in the range from (−15°C) to 76°C. At 20°C it is characterized by the following parameters: viscosity, $\eta$ = 17 mPa·s, dielectric anisotropy $\Delta\varepsilon$ = +9,6, optical anistropy $\Delta n$ = 0,22. A liquid crystalline cell of thickness 10 μm filled by this composition has a threshold voltage $V_{10\%}$ = 1,7, saturation voltage $V_{90\%}$ = 2,4, rise time $T_{on}$ 10—90% = 60 ms, decay time $T_{off}$ 90—10% = 150 ms.

Example 14

A composition in % by weight:

| | |
|---|---|
| 4-(trans-4′-n-propylcyclohexyl)-1-cyanobenzene | 32,28 |
| 4-(trans-4′-n-pentylcyclohexyl)-1-cyanobenzene | 27,70 |
| 4-(trans-4′-n-heptylcyclohexyl)-1-cyanobenzene | 20,2 |
| 1-[p′-(n-hexylbiphenyl)]-2-(p-isothiocyanatophenyl)ethane | 20,00 |

is a nematic in the temperature range from (−15°C) to 66°C. At 20°C it is characterized by the following parameters: wiscosity $\eta$ = 22 mPa·s, $\Delta\varepsilon$ = +9,5, $\Delta n$ = 0,15.
A liquid crystalline cell filled by this compositions shows $V_{10\%}$ = 1,6; $V_{90\%}$ = 2,8; $T_{on}$ 10—90% = 40 ms; $T_{off}$90—10% = 100 ms.

Example 15

A composition in % by weight:

| | |
|---|---|
| 4-(trans-4′-n-propylcyclohexyl)-1-isothiocanatobenzene | 20 |
| 4-(trans-4′-n-hexylcyclohexyl)-1-isothiocanatobenzene | 20 |
| 1-(trans-4-n-pentylcyclohexyl)-1-(p-isothiocanatophenyl)ethane | 15 |
| 4-n-propyl-4′-isothiocyanatobiphenyl | 10 |
| 1-(p′-n-pentylphenyl)-2-[trans-4-(p-isothiocyanatophenyl)cyclohexyl]ethane | 10 |
| 1-(trans-4-n-pentyl cyclohexyl)-2-(p-isothiocyanatobiphenyl)ethane | 7,5 |
| 1-[p′-(trans-4-n-hexylcyclohexyl)phenyl]-2-(p-isothiocyanatophenyl)ethane | 10 |
| 1-(p′-n-pentylbiphenyl)-2-(p-isothiocyanatophenyl)ethane | 7 |
| optically active p′-(2-methylbutyloxy)phenyl-p-octyloxybenzoate | 0,5 |

is nematic in the temperature range from (−30°C) to 77°C. At 25°C it is characterized by the following parameters: viscosity, $\eta$ = 13,7 mPa·s dielectric anisotropy, $\Delta\varepsilon = \varepsilon_{\parallel} - \varepsilon_{\perp}$ = 8,6.

**Claims**

1. Liquid crystalline ethane derivatives expressed by the general formula 1:

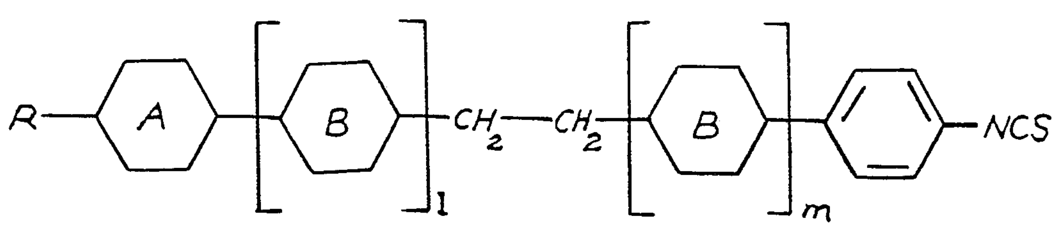

1

where the rings A and B are different or identical and when ring A indicates 1,4-substituted benzene ring then ring B indicates 1,4-substituted benzene ring or 1,4-trans-substituted cyclohexane ring or 1,4-substituted bicyclo[2,2,2]octane ring or 2,5-substituted pyrimidine ring or 2,5-substituted 1,3-dioxane ring, or when ring B indicates 1,4-substituted benzene ring then ring A indicates 1,4-trans-substituted cyclohexane ring or 1,4-substituted bicyclo[2,2,2]octane ring or 2,5-substituted pyrimidine ring or 2,5-substituted 1,3-dioxane ring and R indicates a normal alkyl chain $C_nH_{2n+1}$ or a non-branched alkoxy group $C_nH_{2n+1}O$ or an alkylcarboxylic group $C_nH_{2n+1}COO$ or an alkylcarbonate group $C_nH_{2n+1}OCOO$ or a branched alkyl chain $CH_3—CH_2—CH(CH_3)—(CH_2)_k$ or a branched alkoxy group $CH_3—CH_2—CH(CH_3)—(CH_2)_k—O$, where n is an integer number and assumes values from 1 to 12, k is an integer number and assumes values from 1 to 3 and l and m assume values 1 or zero, where when l=0 and m=0 ring A indicates only the 1,4-substituted bicyclo[2,2,2]octane ring, and when l=0 and m=1, then ring A indicates only the 1,4-substituted benzene ring or 1,4-substituted bicyclo[2,2,2]octane ring or 2,5-substituted pyrimidine ring or 2,5-substituted 1,3-dioxane ring.

2. Liquid crystalline ethane derivatives as claimed in claim 1 where l=0 and m=0 expressed by general formula 1a:

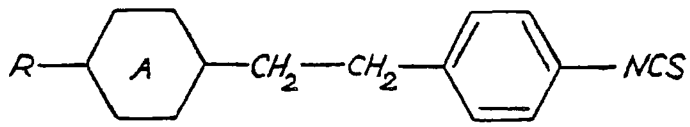

1a

wherein ring A indicates only the 1,4-substituted bicyclo[2,2,2]octane ring.

3. Liquid crystalline ethane derivatives as claimed in claim 1 where l=0 and m=1 expressed by general formula 1b:

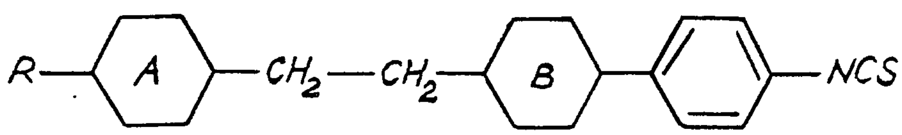

1b

wherein, when ring A indicates 1,4-substituted benzene ring then ring B indicates 1,4-substituted benzene ring or 1,4-substituted bicyclo[2,2,2]octane ring or 1,4-trans-substituted cyclohexane ring or 2,5-substituted pyrimidines ring or 2,5-substituted 1,3-dioxane ring and when ring B indicates 1,4-substituted benzene ring then ring A indicates 1,4-substituted bicyclo[2,2,2]octane ring or 2,5-substituted pyrimidine ring or 2,5-substituted 1,3-dioxane ring.

4. Liquid crystalline ethane derivatives as claimed in claim 1 where l=1 and m=0 expressed by general formula 1c:

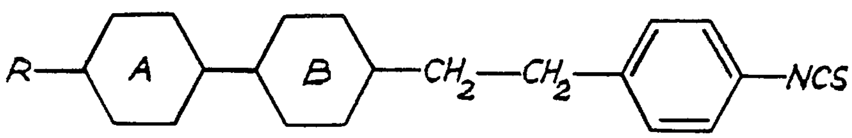

1c

where in when ring A indicates 1,4-substituted benzene ring then ring B indicates 1,4-substituted benzene ring or 1,4-trans-substituted cyclohexane ring or 1,4-substituted bicyclo[2,2,2]octane ring or 2,5-substituted pyrimidine ring or 2,5-substituted 1,3-dioxane ring and when ring B indicates 1,4-substituted benzene ring, then ring A indicates 1,4-trans-substituted cyclohexane ring or 2,5-substituted pyrimidine ring, or 1,4-substituted bicyclo[2,2,2]octane ring or 2,5-substituted 1,3-dioxane ring.

5. Method of preparation of liquid crystalline ethane derivatives expressed by formula 1 as defined in claim 1 specific in that compound of general formula 2:

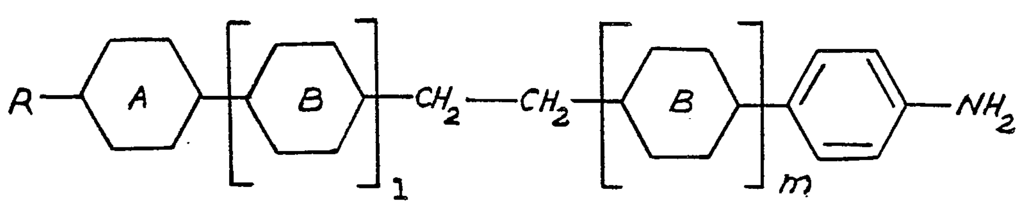

2

where all the notations are the same as in formula 1, is treated by thiophosgene in an organic solvent preferably chloroform in the presence of a basic compound preferably sodium bicarbonate or calcium carbonate or carbon disulphide in an organic solvent, preferably ether in the presence of dicyclohexylcarbodiimide or by carbon disulphide in a solvent, in the presence of a tertiary amine, and the obtained trialkylammonium dithiocarbamate salt is separated from the solution and then dissolved in an organic solvent preferably chloroform and then treated by an acid chloride preferably ethyl chloroformate in the presence of a tertiary amine preferably triethylamine.

6. Liquid crystalline compositions being a mixture of at least two liquid crystalline components and of optically active compounds and/or dyes specific in that at least one liquid crystalline compound belongs to the group of compounds in claim 1 in a quantity above 2% by weight.

7. Compositions as claimed in claim 6 specific in that they include at least one liquid crystalline compound of formula 1 in a quantity of 5—40% by weight.

8. Compositions as claimed in claim 6 and 7 specific in that beside at least one liquid crystalline compound from the group of compounds in claim 1 they include at least one compound of the known liquid crystalline compounds with the isothiocyanato group expressed by formulae I—VII,

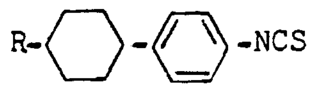
I

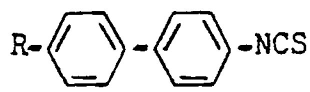
II

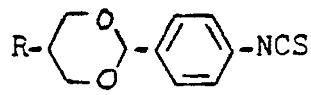
III

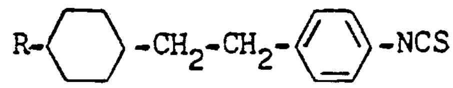
IV

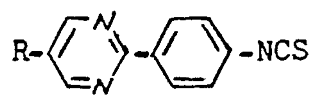
V

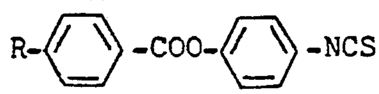
VI

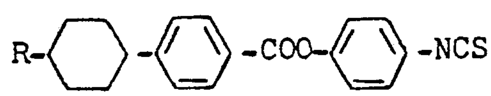
VII

where R indicates a normal or a branched alkyl group containing from 3 to 10 carbon atoms in the alkyl chain.

9. Compositions as claimed in claim 8 specific in that the known liquid crystalline compounds are 4-(trans-4'-n-alkylcyclohexyl)-1-isothiocyanatobenzenes especially in the form of 4-(trans-4'-n-propyl-cyclohexyl)-1-isothiocyanatobenzene and/or 4-(trans-4'-n-hexylcyclohexyl)-1-isothiocyanatobenzene.

10. Compositions claimed as in claim 8 or 9 specific in that the known liquid crystalline compounds are 4-(trans-4'-n-alkylcyclohexyl)-1-isothiocyanatobenzenes and/or 4-alkyl-4'-isothiocyanatobiphenyls.

11. Compositions claimed as in claim 6 or 7 specific in that besides at least one liquid crystalline compound from the group of compounds in claim 1 they include at least one from the known liquid crystalline compounds expressed by formulae VIII—XXIV,

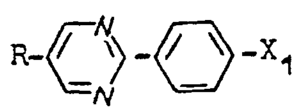
VIII

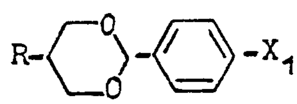
IX

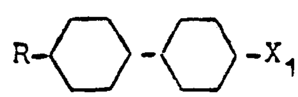
X

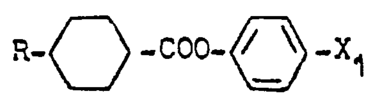
XI

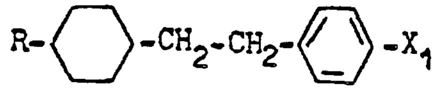 XII

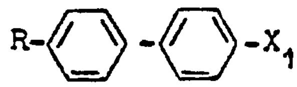 XIII

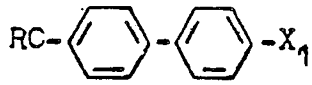 XIV

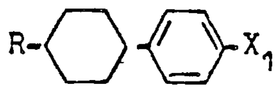 XV

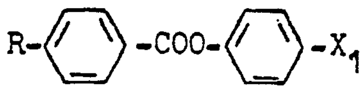 XVI

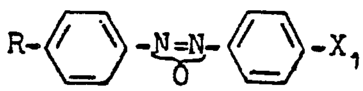 XVII

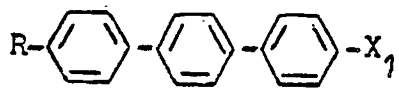 XVIII

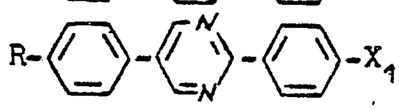 XIX

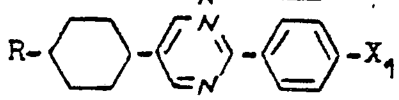 XX

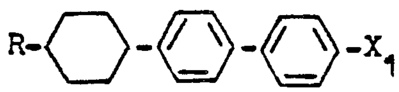 XXI

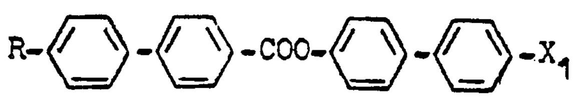 XXII

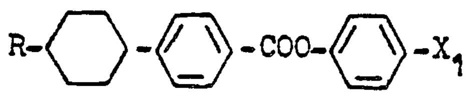 XXIII

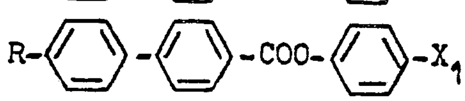 XXIV

where R indicates the alkyl group and $X_1$ the alkyl, cyano or alkoxy group.

12. Compositions claimed in claim 6 or 7 specific in that besides at least one liquid crystalline compound from the group of compounds from claim 1 they include simultaneously at least one compound from claim 8 and 11.

13. In the liquid crystalline cell containing a liquid crystalline mixture improvement specific in that the composition includes a compound from claim 1.

**Patentansprüche**

1. Flüssigkristall-Äthanderivate wie in der allgemeinen Formel 1 dargestellt,

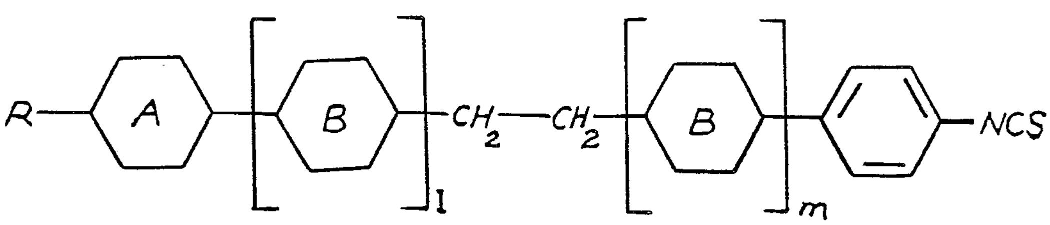

1

worin die Ringe A und B verschieden oder identisch sind, und, wenn Ring A einen 1,4-substituierten Benzolring darstellt, Ring B einen 1,4-substituierten Benzolring, oder einem 1,4-trans-substituierten Cyclohexanring, oder einen 1,4-substituierten Bicyclo[2,2,2]octanring, oder einen 2,5-substituierten Pyrimidinring, oder einen 2,5-substituierten 1,3-Dioxanring darstellt, oder, wenn Ring B einen 1,4-substituierten Benzolring darstellt, Ring A einen 1,4-trans-substituierten Cyclohexanring, oder einen 1,4-substituierten Bicyclo[2,2,2]octanring, oder einen 2,5-substituierten Pyrimidinring oder einen 2,5-substituierten 1,3-Dioxanring darstellt, und R eine normale Alkylkette $C_nH_{2n+1}$ oder eine nicht verzweigte Alkoxygruppe $C_nH_{2n+1}O$, oder eine Alkylcarboxylgruppe $C_nH_{2n+1}COO$, oder eine Alkylcarbonatgruppe $C_nH_{2n+1}$ OCOO, oder eine verzweigte Alkylgruppe $CH_3—CH_2—CH(CH_3)—(CH_2)_k$, oder eine verzweigte Alkoxygruppe $CH_3—CH_2—CH(CH_3)—(CH_2)_k—O$ darstellt, worin n eine ganze Zahl ist und Werte von 1 bis 12 bedeutet, k eine ganze Zahl ist und Werte von 1 bis 3 bedeutet und l und m Werte von 1 oder 0 bedeuten, worin, wenn l=0 und m=0 ist, Ring A nur den 1,4-substituierten Bicyclo[2,2,2]octanring darstellt, und, wenn l=0 und m=1, Ring A nur den 1,4-substituierten Benzolring, oder den 1,4-substituierten Bicyclo-[2,2,2]octanring oder den 2,5-substituierten Pyrimidinring, oder den 2,5-substituierten 1,3-Dioxanring darstellt.

2. Flüssigkristall-Äthanderivate nach Anspruch 1, worin l=0 und m=0, wie in der allgemeinen Formel 1a dargestellt,

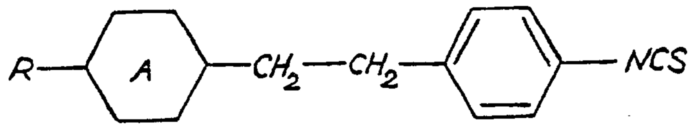

1a

worin Ring A nur den 1,4-substituierten Bicyclo[2,2,2]octanring darstellt.

3. Flüssigkristall-Äthanderivate nach Anspruch 1, worin l=0 und m=1, wie in der allgemeinen Formel 1b dargestellt,

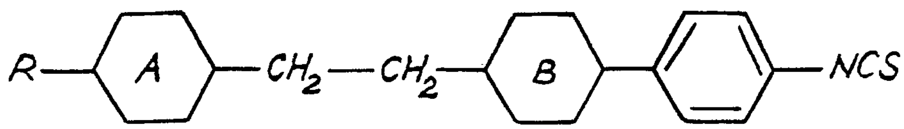

1b

worin, wenn Ring A einen 1,4-substituierten Benzolring darstellt, Ring B einen 1,4-substituierten Benzolring, oder einen 1,4-substituierten Bicyclo[2,2,2]octanring, oder einen 1,4-trans-substituierten Cyclohexanring, oder einen 2,5-substituierten Pyrimidinring, oder einen 2,5-substituierten 1,3-Dioxanring darstellt, und, wenn Ring B einen 1,4-substituierten Benzolring darstellt, Ring A einen 1,4-substituierten Bicyclo-[2,2,2]octanring, oder einen 2,5-substituierten Pyrimidinring oder einen 2,5-substituierten 1,3-Dioxanring dargestellt.

4. Flüssigkristall-Äthanderivate nach Anspruch 1, worin l=1 und m=0, wie in der allgemeinen Formel 1c dargestellt,

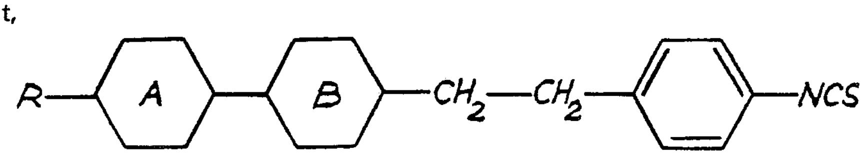

1c

worin, wenn Ring A einen 1,4-substituierten Benzolring darstellt, Ring B einen 1,4-substituierten Benzolring, oder einen 1,4-trans-substituierten Cyclohexanring, oder einen 1,4-substituierten Bicyclo-[2,2,2]octanring, oder einem 2,5-substituierten Pyrimidinring, oder einen 2,5-substituierten 1,3-Dioxanring darstellt, und, wenn Ring B einen 1,4-substituierten Benzolring darstellt, Ring A einen 1,4-trans-substituierten Cyclohexanring, oder einen 1,4-substituierten Bicyclo[2,2,2]octanring, oder einen 2,5-substituierten Pyrimidinring oder einen 2,5-substituierten 1,3-Dioxanring darstellt.

5. Verfahren zur Herstellung von Flüssigkristall-Äthanderivaten nach der allgemeinen Formel 1 wie in Anspruch 1 beschrieben, dadurch gekennzeichnet, daß die Verbindung der allgemeinen Formel 2

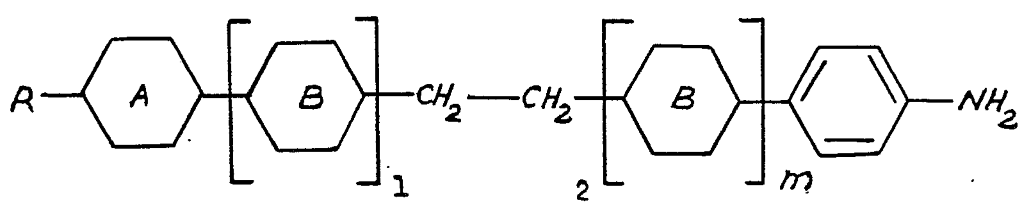

wobei sämtliche Benzeichnungen dieselben wie in Formel 1 sind, mit Thiophosgen in einem organischen Lösungsmittel behandelt wird, vorzugsweise Chloroform in Anwesenheit einer Basisverbindung, vorzugsweise Natriumbicarbonat oder Kalziumcarbonat oder Schwefelkohlenstoff in einem organischen Lösungsmittel, vorzugsweise Äther in der Anwesenheit von Dicyclohexylcarbondiimid oder mit Schwefelkohlenstoff in einem Lösungsmittel, in Anwesenheit eines tertiären Amins, und das erhaltene Trialkylammoniumdithiocarbamatsalz von der Lösung abgetrennt und dann in einem organischen Lösungsmittel, vorzugsweise Chloroform, aufgelöst wird, und dann mit einem Säurechlorid, vorzugsweise Ethylchlorkohlensäureester, in Anwesenheit eines tertiären Amins, vorzugsweise Triethylamin, behandelt wird.

6. Flüssigkristall-Zusammensetzungen, welche eine Mischung von wenigstens zwei Flüssigkristall-Bestandteilen und wahlweise aktiven Verbindungen und/oder Farbstoffen darstellen, dadurch gekennzeichnet, daß wenigstens eine Flüssigkristall-Verbindung zu der Gruppe der Verbindungen nach Anspruch 1 gehört, in einer Menge von über 2 Gewichtsprozent.

7. Zusammensetzungen nach Anspruch 6, dadurch gekennzeichnet, daß sie wenigstens eine Flüssigkristall-Verbindung nach Formel 1, in einer Menge von 5 bis 40 Gewichtsprozent enthalten.

8. Zusammensetzungen nach den Ansprüchen 6 und 7, dadurch gekennzeichnet, daß sie neben wenigstens einer Flüssigkristall-Verbindung aus der Gruppe der Verbindungen nach Anspruch 1, wenigstens eine Verbindung der bekannten Flüssigkristall-Verbindung mit den Isothiocyanatgruppen wie in den Formeln I bis VII dargestellt, enthalten,

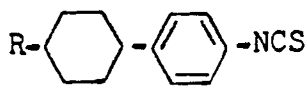 I

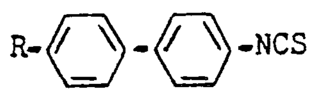 II

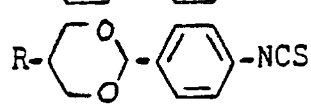 III

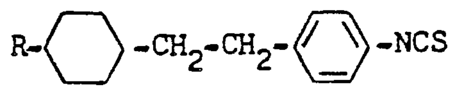 IV

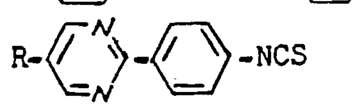 V

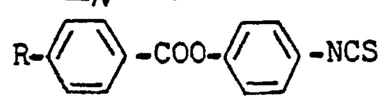 VI

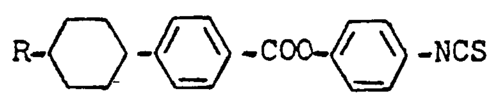 VII

worin R eine normale oder eine verzweigte Alkylgruppe mit 3 bis 10 Kohlenstoffatomen in der Alkylkette darstellt.

9. Zusammensetzungen nach Anspruch 8, dadurch gekennzeichnet, daß die bekannten Flüssigkristall-Verbindungen 4-(trans-4′-n-alkylcyclohexyl)-1-isothiocyanatobenzole sind, insbesondere in der Form von 4-(trans-4′n-propylcyclohexyl)-1-isothiocyanatobenzol und/oder 4-(trans-4′-n-hexylcyclohexyl)-1-isothiocyanatobenzol.

10. Zusammensetzungen nach den Ansprüchen 8 oder 9, dadurch gekennzeichnet, daß die bekannten Flüssigkristall-Verbindungen 4-(trans-4′-n-alkylcyclohexyl)-1-isothiocyanatobenzole und/oder 4-alkyl-4′-isothiocyanatobiphenyle sind.

11. Zusammensetzungen nach den Ansprüchen 6 oder 7, dadurch gekennzeichnet, daß neben wenigstens einer Flüssigkristall-Verbindung aus der Gruppe der Verbindungen nach Anspruch 1, wenigstens eine der bekannten Flüssigkristall-Verbindungen, wie in den Formeln VIII bis XXIV dargestellt, enthalten,

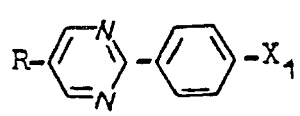 VIII

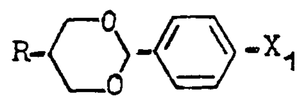 IX

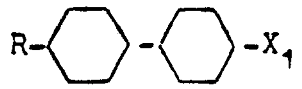
X

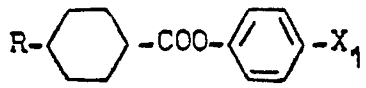
XI

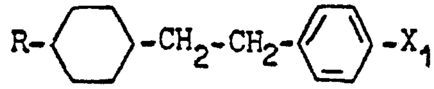
XII

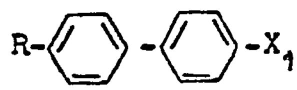
XIII

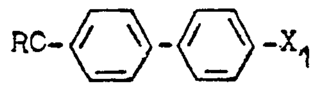
XIV

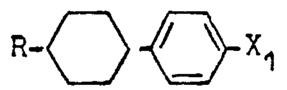
XV

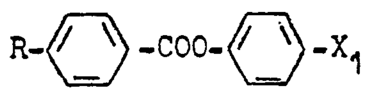
XVI

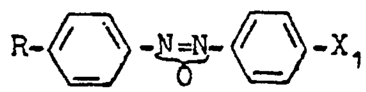
XVII

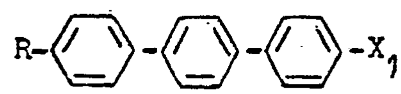
XVIII

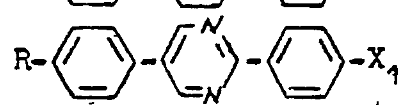
XIX

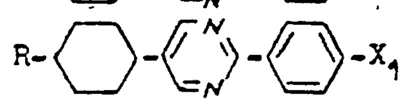
XX

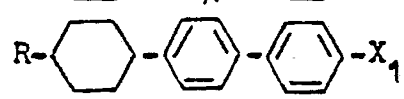
XXI

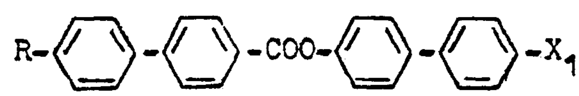
XXII

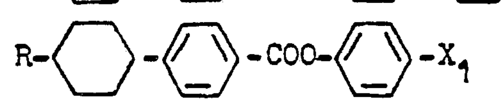
XXIII

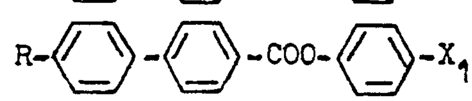
XXIV

worin R die Alkylgruppe, und $X_1$ die Alkyl-, Cyano- oder Alkoxygruppe angeben.

12. Zusammensetzungen nach den Ansprüchen 6 oder 7, dadurch gekennzeichnet, daß neben wenigstens einer Flüssigkristall-Verbindung aus der Gruppe der Verbindungen nach Anspruch 1, gleichzeitig wenigstens eine Verbindung nach den Ansprüchen 8 und 11 enthalten ist.

13. Flüssigkristall-Zelle mit einer Flüssigkristall-Mischung, wobei die Verbesserung dadurch gekennzeichnet ist, daß die Zusammensetzung eine Verbindung nach Anspruch 1 enthält.

**Revendications**

1. Dérivés d'éthane formant des cristaux liquides répondant à la formule générale 1:

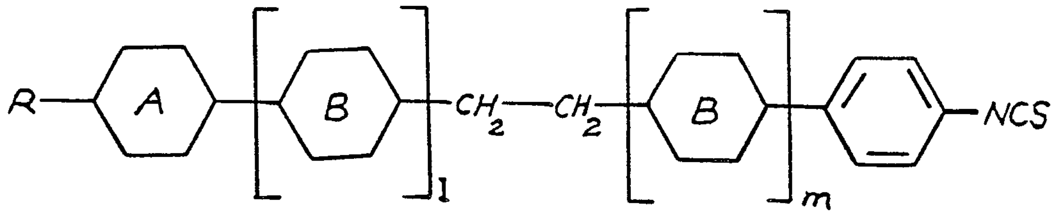

1

dans laquelle les cycles A et B sont différents ou identiques et, lorsque le cycle A est un cycle benzène 1,4-substitué, le cycle B est un cycle benzène 1,4-substitué ou un cycle cyclohexane 1,4-trans-substitué ou un cycle bicyclo[2.2.2]octane 1,4-substitué ou un cycle pyrimidine 2,5-substitué ou un cycle 1,3-dioxanne 2,5-substitué ou, lorsque le cycle B représente un cycle benzène 1,4-substitué, le cycle A représente un cycle cyclohexane 1,4-trans-substitué ou un cycle bicyclo[2.2.2]octane 1,4-substitué ou un cycle pyrimidine 2,5-substitué ou un cycle 1,3-dioxanne 2,5-substitué et R représente une chaîne alcoyle normale $C_nH_{2n+1}$ ou un groupe alcoxy non ramifié $C_nH_{2n+1}O$ ou un groupe alcoylcarboxylique $C_nH_{2n+1}COO$ ou un groupe alcoylcarbonato $C_nH_{2n+1}OCOO$ ou un chaîne alcoyle ramifiée $CH_3—CH_2—CH(CH_3)—(CH_2)_k$ ou un groupe alcoxy ramifié $CH_3—CH_2—CH(CH_3)—(CH_2)_k—O$, où n est un nombre entier ayant une valeur de 1 à 12, k est un nombre entier ayant une valeur de 1 à 3 et 1 et m ont pour valeurs 1 ou 0, où lorsque l=0 et m=0, le cycle A représente uniquement un cycle bicyclo[2.2.2]octane 1,4-substitué et lorsque l=0 et m=1, le cycle A représente uniquement un cycle benzène 1,4-substitué ou un cycle bicyclo[2.2.2]octane 1,4-substitué ou un cycle pyrimidine 2,5-substitué ou un cycle 1,3-dioxanne 2,5-substitué.

2. Dérivés de l'éthane formant des cristaux liquides selon la revendication 1 où l=0 et m=0, représentés par la formule générale 1a:

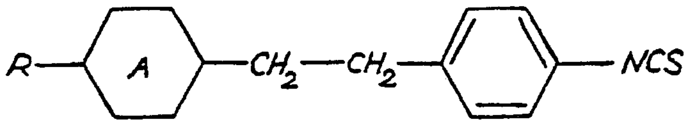

1a

dans laquelle le cycle A représente uniquement un cycle bicyclo[2.2.2]octane 1,4-substitué.

3. Dérivés d'éthane formant des cristaux liquides selon la revendication 1 où l=0 et m=1, représentés par la formule générale 1b:

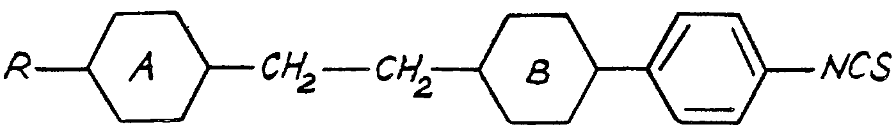

1b

dans laquelle, lorsque le cycle A représente un cycle benzène 1,4-substitué, le cycle B représente un cycle benzène 1,4-substitué ou un cycle bicyclo[2.2.2]octane 1,4-substitué ou un cycle cyclohexane 1,4-trans-substitué ou un cycle pyrimidine 2,5-substitué ou un cycle 1,3-dioxanne 2,5-substitué ou, lorsque le cycle B représente un cycle benzène 1,4-substitué, le cycle A représente un cycle bicyclo[2.2.2]octane 1,4-substitué ou un cycle pyrimidine 2,5-substitué ou un cycle 1,3-dioxanne 2,5-substitué.

4. Dérivés de l'éthane formant des cristaux liquides selon la revendication 1 où l=1 et m=0, représentés par la formule générale 1c:

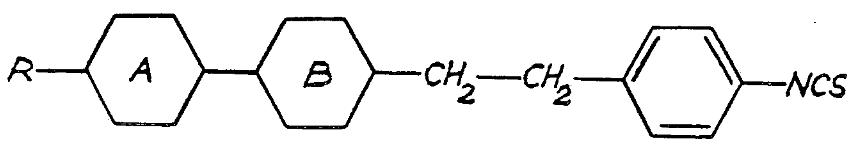

1c

dans laquelle, lorsque le cycle A représente un cycle benzène 1,4-substitué, le cycle B représente un cycle benzène 1,4-substitué ou un cycle cyclohexane 1,4-trans-substitué ou un cycle bicyclo[2.2.2]octane 1,4-substitué ou un cycle pyrimidine 2,5-substitué ou un cycle 1,3-dioxanne 2,5-substitué et, lorsque le cycle B représente un cycle benzène 1,4-substitué, le cycle A représente un cycle cyclohexane 1,4-trans-substitué ou un cycle bicyclo[2.2.2]octane 1,4-substitué ou un cycle pyrimidine 2,5-substitué ou un cycle 1,3-dioxanne 2,5-substitué.

5. Procédé de préparation de dérivés de l'éthane formant des cristaux liquides représentés par la formule 1 comme défini dans la revendication 1, caractérisé en ce que l'on traite un composé de formule générale 2:

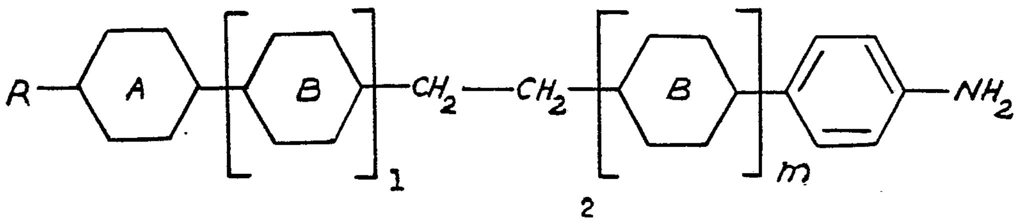

2

dans laquelle toutes les notations sont les mêmes que pour la formule 1, avec du thiophosgène dans un solvant organique, de préférence le chloroforme, en présence d'un composé basique, de préférence le bicarbonate de sodium ou le carbonate de calcium ou le disulfure de carbone, dans un solvant organique, de préférence l'éther en présence de dicyclohexylcarbodiimide ou avec du disulfure de carbone dans un solvant, en présence d'une amine tertiaire, puis on sépare de la solution le sel dithiocarbamate de trialcoylammonium obtenu, puis on le dissout dans un solvant organique, de préférence le chloroforme, puis on traite avec un chlorure d'acide, de préférence le chloroformiate déthyle, en présence d'une amine tertiaire, de préférence la triéthylamine.

6. Compositions formant des cristaux liquides qui sont un mélange d'au moins deux composants formant des cristaux liquides et de composés optiquement actifs et/ou de colorants, caractérisées en ce qu'au moins un composé formant des cristaux liquides appartient au groupe des composés de la revendication 1 en une quantité supérieure à 2% en poids.

7. Compositions selon la revendication 6, caractérisées en ce qu'elles comprennent au moins un composé formant des cristaux liquides de formule 1 en une quantité de 5 à 40% en poids.

8. Compositions selon les revendications 6 et 7, caractérisées en ce qu'en plus d'au moins un composé formant des cristaux liquides appartenant au groupe des composés de la revendication 1, elles comprennent au moins un composé choisi parmi les composés connus formant des cristaux liquides ayant un groupe isocyanato représentés par les formules I à VII,

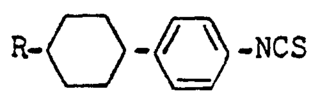

I

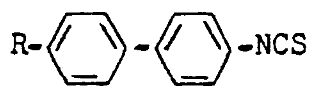

II

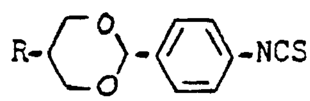

III

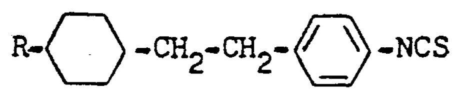

IV

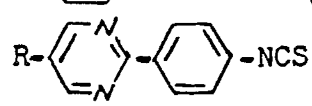

V

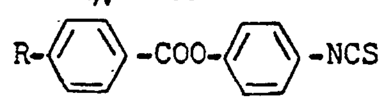

VI

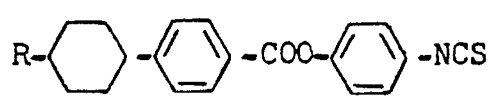

VII

dans lesquelles R représente un groupe alcoyle normal ou ramifié contenant de 3 à 10 atomes de carbone dans la chaîne alcoyle.

9. Compositions selon la revendication 8, caractérisées en ce que les composés connus formant des cristaux liquides sont des 4-(trans-4'-n-alcoylcyclohexyl)-1-isothiocyanatobenzènes en particulier sous forme du 4-(trans-4'-n-propylcyclohexyl)-1-isocyanatobenzène et/ou du 4-(trans-4'-n-hexylcyclohexyl)-1-isothiocyanatobenzène.

10. Compositions selon les revendications 8 ou 9, caractérisées en ce que les composés connus formant des cristaux liquides sont des 4-(trans-4'-n-alcoylcyclohexyl)-1-isothiocyanatobenzènes et/ou des 4-alcoyl-4'-isocyanatobiphényles.

11. Compositions selon les revendications 6 ou 7, caractérisées en ce qu'en plus d'au moins un

composé formant des cristaux liquides appartenant au groupe des composés de la revendication 1, elles comprennent au moins un composé connu formant des cristaux liquides représenté par les formules VIII à XXIV,

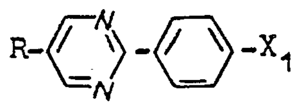 VIII

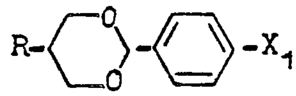 IX

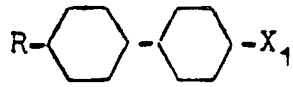 X

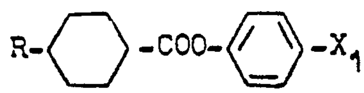 XI

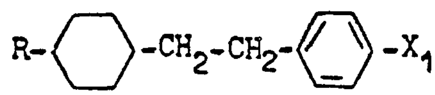 XII

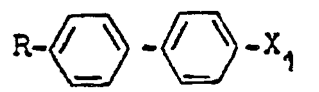 XIII

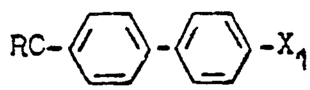 XIV

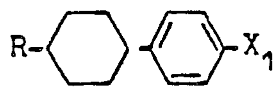 XV

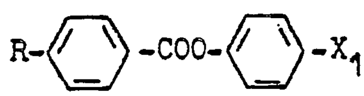 XVI

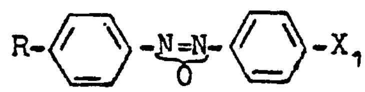 XVII

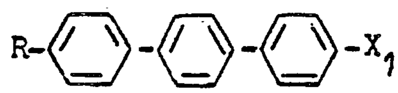 XVIII

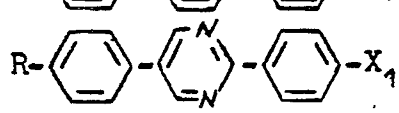 XIX

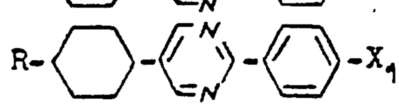 XX

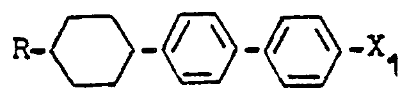 XXI

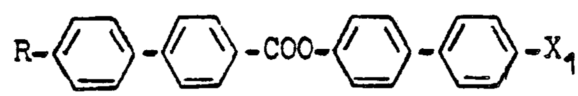 XXII

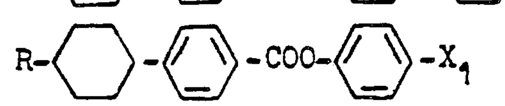 XXIII

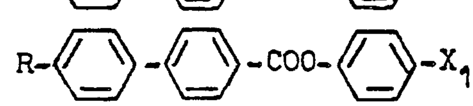 XXIV

dans lesquelles R représente un groupe alcoyle et $X_1$ représente un groupe alcoyle, cyano ou alcoxy.

12. Compositions selon les revendications 6 ou 7, caractérisées en ce qu'en plus d'au moins un

composé formant des cristaux liquides appartenant au groupe des composés de la revendication 1, elles comprennent simultanément au moins un composé selon les revendications 8 et 11.

13. Dans une cellule à cristaux liquides contenant un mélange formant des cristaux liquides, le perfectionnement caractéristique consistant en ce que la composition comprend un composé selon la revendication 1.